# EUROPÄISCHE PATENTSCHRIFT

(11) **EP 2 029 600 B1**
(45) Veröffentlichungstag und Bekanntmachung des Hinweises auf die Patenterteilung: **14.03.2012**
(21) Anmeldenummer: 07729158.1
(22) Anmeldetag: 15.05.2007
(51) Int. Cl.: C07D 475/08, A61K 31/495, A61P 11/00, A61P 17/00, A61P 25/00, A61P 29/00, A61P 35/00

(54) **SUBSTITUIERTE PTERIDINE, DIE MIT EINEM VIERGLIEDRIGEN HETEROCYCLUS SUBSTITUIERT SIND**
SUBSTITUTED PTERIDINES SUBSTITUTED WITH A FOUR-MEMBERED HETEROCYCLE
PTÉRIDINES SUBSTITUÉES PAR UN HÉTÉROCYCLE À QUATRE CHAÎNONS

(30) Priorität: 24.05.2006 EP 06114539
(43) Veröffentlichungstag der Anmeldung: 04.03.2009
(73) Patentinhaber: Boehringer Ingelheim International GmbH, 55216 Ingelheim am Rhein (DE); Boehringer Ingelheim Pharma GmbH & Co. KG, 55216 Ingelheim am Rhein (DE)
(72) Erfinder: DOLLINGER, Horst, 88433 Schemmerhofen (DE); MARTYRES, Domnic, 88400 Biberach (DE); GOEGGEL, Rolf, 89073 Ulm (DE); JUNG, Birgit, 88471 Laupheim (DE); NICKOLAUS, Peter, 88447 Warthausen (DE)
(74) Vertreter: Hammann, Heinz
(86) Internationale Anmeldenummer: PCT/EP2007/054709
(87) Internationale Veröffentlichungsnummer: WO 2007/135027

(56) Entgegenhaltungen:
- EP-A- 0 134 922
- DE-A1- 3 540 952
- MERZ K-H ET AL: "Synthesis of 7-Benzylamino-6-chloro-2-piperazino-4-pyrr olidinopterid ine and Novel Derivatives Free of Positional Isomers. Potent Inhibitors of cAMP-Specific Phosphodiesterase and of Malignant Tumor Cell Growth" JOURNAL OF MEDICINAL CHEMISTRY, AMERICAN CHEMICAL SOCIETY. WASHINGTON, US, Bd. 41, Nr. 24, 1998, Seiten 4733-4743, XP002239611 ISSN: 0022-2623 in der Anmeldung erwähnt

## Beschreibung

Die Erfindung betrifft neue Pteridine, die geeignet sind zur Behandlung von
- Atemwegs- oder gastrointestinalen Beschwerden oder Erkrankungen,
- entzündlichen Erkrankungen der Gelenke, der Haut oder der Augen,
- Erkrankungen des periphären oder zentralen Nervensystems oder
- Krebserkrankungen,
sowie pharmazeutische Zusammensetzungen die diese Verbindungen beinhalten.

### STAND DER TECHNIK

Pteridine sind als Wirkstoffe mit antiproliferativer Wirkung aus dem Stand der Technik bekannt. Merz et al. beschreiben im Journal of Medicinal Chemistry 1998, 41, 4733-4743 die Herstellung von 7-Benzylamino-6-chlor-2-piperazino-4- pyrrolidinopteridin und Derivaten davon, welche frei von Stellungsisomeren sind. Es wurde gezeigt, dass die hergestellten Verbindungen das Wachstum von Tumorzellen hemmen können. In der DE 3540952 werden 2-Piperazino-pteridine beschrieben, die in der 6-Stellung mit einem Halogenatom, ausgewählt aus einem Fluor-, Chlor- oder Bromatom, substituiert sind. Es wurde gezeigt, dass diese Verbindungen die Aktivität von Tumorzellen und von Humanthrombozyten in vitro hemmen konnten. Die DE 3323932 offenbart 2-Piperazino-pteridine, die in der 4-Stellung eine Dialkylamino-, Piperidino-, Morpholino-, Thiomorpholino- oder 1-Oxidothiomorpholinogruppe tragen. Es wurde gezeigt, dass diese Verbindungen die Aktivität von Tumorzellen und von Humanthrombozyten in vitro hemmen konnten. In der DE 3445298 werden Pteridine mit einer großen Anzahl an unterschiedlichen Substituenten in der 2-, 4-, 6- und 7-Stellung beschrieben, wobei sich Verbindungen mit einer 2-Piperazinogruppe am Pteridingerüst als Hemmstoffe für das Tumorwachstum eignen sowie antithrombotische und metastasenhemmende Eigenschaften aufweisen. In der US 2,940,972 werden tri- und tetrasubstituierte Pteridinderivate offenbart, wobei allgemein Angaben gemacht werden, dass diese Pteridine wertvolle pharmakologische Eigenschaften, nämlich coronarerweiternde, sedative, antipyretische und analgetische Wirkungen aufweisen.

Die aus dem Stand der Technik bekannten Phosphodiesterase 4 Inhibitoren sind bekannt dafür Nebenwirkungen wie Übelkeit und Erbrechen auszulösen (Doherty, 1999,Curr. Op. Chem. Biol., Aug. 3, (4):466-73). Die in dieser Erfindung benannten Substanzen hemmen bevorzugt die B-Isoenzyme der Phosphodiesterase 4, sind also bevorzugte PDE4B-Inhibitoren und sind infolgedessen besonders bevorzugt zur Behandlung der genannten Erkrankungen geeignet, da sie in einem Tiermodell für Übelkeit und Erbrechen (S. Murinus, Yamamoto K. et al.,Physiol. Behav., 2004, Oct. 30, 83(1), 151-6) diese Nebenwirkungen im Gegensatz zu anderen PDE4-inhibitoren, die vorzugsweise die anderen PDE4-Isoenzyme (z.B. Isoenzyme A, C oderD) hemmen, nicht auslösen.

Aufgabe der vorliegenden Erfindung ist es neue Verbindungen bereit zu stellen, die geeignet sind zur Vorbeugung oder Behandlung von Atemwegs- oder gastrointestinalen Beschwerden oder Erkrankungen, entzündlichen Erkrankungen der Gelenke, der Haut oder der Augen, Erkrankungen des periphären oder zentralen Nervensystems, oder Krebserkrankungen, insbesondere solche Verbindungen, die durch geringere Nebenwirkungen, insbesondere Emesis und Nausea, gekennzeichnet sind.

### BESCHREIBUNG DER ERFINDUNG

Überraschenderweise konnte nun gefunden werden, dass Pteridine der Formel **1** geeignet sind zur Behandlung entzündlicher Erkrankungen.

Gegenstand der vorliegenden Erfindung sind deshalb Verbindungen der Formel **1**, 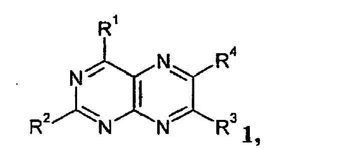 worin
- R¹: ein gesättigter oder ungesättigter, viergliedriger Heterocyclus, der ein Stickstoffatom enthält und gegebenenfalls ein weiteres Atom ausgewählt aus der Gruppe bestehend aus Stickstoff, Schwefel und Sauerstoff enthalten kann;
- R²: Halogen, OR^{2.1}, SR^{2.1}, NR^{2.1}R^{2.2},
wobei
R^{2.1} H, C₁₋₄-Alkyl, C₆-₁₀-Aryl, C₇₋₁₁-Aralkyl;
R^{2.2} H, C₁₋₄-Alkyl, C₆₋₁₀-Aryl, C₇₋₁₁- Aralkyl; oder
- R²: ein Rest ausgewählt aus der Gruppe bestehend aus C₆₋₁₀-Aryl, C₅₋₁₀-Heleroaryl und einem fünf-, sechs- oder siebengliedrigen Heterocyclus, der ein Stickstoffatom enthält und der gegebenenfalls ein weiteres Atom ausgewählt aus der Gruppe bestehend aus Stickstoff, Schwefel und Sauerstoff enthalten kann, wobei dieser Rest gegebenenfalls mit einem Rest ausgewählt aus der Gruppe bestehend aus C₁₋₆-Alkyl, O-C₁₋₆-Alkyl, C₃₋₆-Cycloalkyl, C₇₋₁₁- Aralkyl und N(C₁₋₄-Alkyl)₂ substituiert sein kann
- R³: eine Gruppe der Formel **1a**, 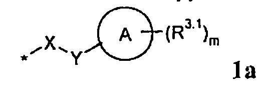
worin
- A: ein Ring ausgewählt aus der Gruppe bestehend aus einem monocyclischen, heterocyclischen Ring, einem bicyclischen Ring, der gegebenenfalls ein oder mehrere Heteroatome enthält, einem C₆₋₁₀-Aryl und einem C₅₋₁₀-Heteroaryl,
- X: NR^{3.2}, O, S;
- Y: C₁₋₄-Alkylen, der gegebenenfalls mit einem oder mehreren R^{3.3} substituiert sein kann,
- m: 0, 1, 2 oder 3
- R^{3.1}: jeweils unabhängig voneinander C₁₋₆-Alkyl, C₆₋₁₀-Aryl, COOR^{3.1.1}, CONR^{3.1.1}R^{3.1.2}, CN, NR^{3.1.1}R^{3.12}, NHCOR^{3.1.1}, OR^{3.1.1}, O-C₁₋₆-Haloalkyl, SO₂R^{3.1.1}, SO₂NH₂, Halogen, C₁₋₆-Haloalkyl, C₁₋₆-alkyl-CONR^{3.1.1}R^{3.1.2}, C₁₋₆-alkyl-NR^{3.1.1}R^{3.1.2}, C₁₋₆-alkyl-CONH₂, O-C₁₋₆-alkylen-NH_{2,} O-C₃₋₆-Cycloalkyl, O-C₁₋₄-Alkylen-C₃₋₆-cycloalkyl,O-C₁₋₄ -Alkylen-CONH₂, SO₂NR^{3.1.1}R^{3.1.2.}, oder
- R^{3.1}: bildet gemeinsam mit zwei Atomen von A einen 5 oder 6-gliedrigen carbocyclischen Ring oder einen 5 oder 6-gliedrigen heterocyclischen Ring, der gegebenenfalls ein oder mehrere Heteroatome ausgewählt aus der Gruppe bestehend aus Sauerstoff und Stickstoff enthalten kann,
worin
R^{3.1.1} H, C₁₋₆-Alkyl;
R^{3.1.2} H, C₁₋₆-Alkyl; und
- R^{3.2}: H, C₁₋₆-Alkyl;
und worin
- R^{3.3} oder: jeweils unabhängig voneinander H, C₁₋₆-Alkyl, C₁₋₆-Alkyl-OH, C₃₋₆-Cycloalkyl, C₃₋₆-Cycloalkyl-OH, O-C₁₋₆-Alkyl, COOR^{3.1.1}, COO-C₁₋₆-Alkyl, CONR^{3.1.1}R^{3.1.2} ;
oder
- R^{3.3}: bildet gemeinsam mit einem oder zwei Kohlenstoffatomen von Y einen carbocyclischen Ring mit 3, 4, 5 oder 6 Kohlenstoffatomen,

- R⁴: ein Rest ausgewählt aus der Gruppe bestehend aus Halogen, C₁₋₆-Alkyl, C₂₋₆-Alkenyl, C₂₋₆-Alkinyl, C₃₋₆-Cycloalkyl, C₃₋₆-Cycloalkenyl, OR^{4.1}, SR^{4.1}, C₁₋₆-Haloalkyl, NR^{4.1}R^{4.2} oder ein Rest ausgewählt aus der Gruppe bestehend aus C₆₋₁₀-Aryl, 3-10-gliedriger Heterocyclus und C₅₋₁₀-Heteroaryl, der gegebenenfalls mit einem oder mehreren Resten ausgewählt aus der Gruppe bestehend aus C₁₋₆-Alkyl, C₁₋₆-Haloalkyl, CN, O-C₁₋₆-Alkyl, Halogen substituiert sein kann,
R^{4.1} H, C₁₋₆-Alkyl, C₆₋₁₀-Aryl, C₇₋₁₁- Aralkyl;
R^{4.2} H, C₁₋₆-Alkyl, C₆₋₁₀-Aryl, C₇₋₁₁- Aralkyl;
bedeuten, sowie pharmakologisch verträgliche Salze, Diastereomere, Enantiomere, Racemate, Hydrate oder Solvate davon.

Ein weiterer Gegenstand der Erfindung sind die oben genannten Verbindungen der Formel **1**, wobei R², R³ und R⁴ die obigen Definitionen besitzen und wobei
- R¹: ein Azetidinring
bedeutet, sowie pharmakologisch verträgliche Salze, Diastereomere, Enantiomere, Racemate, Hydrate oder Solvate davon.

Ein weiterer Gegenstand der Erfindung sind die oben genannten Verbindungen der Formel **1**, wobei R³ und R⁴ die obigen Definitionen besitzen und wobei
- R¹: ein Azetidinring,
- R²: ein fünf- oder sechsgliedriger Heterocyclus, der ein oder zwei Stickstoffatome enthält;
bedeuten, sowie pharmakologisch verträgliche Salze, Diastereomere, Enantiomere, Racemate, Hydrate oder Solvate davon.

Ein weiterer Gegenstand der Erfindung sind die oben genannten Verbindungen der Formel **1**, wobei R³ und R⁴ die obigen Definitionen besitzen und wobei
- R¹: Azetidinring;
- R²: ein sechsgliedriger Heterocyclus, der zwei Stickstoffatome enthält;
bedeuten, sowie pharmakologisch verträgliche Salze, Diastereomere, Enantiomere, Racemate, Hydrate oder Solvate davon.

Weiterhin bevorzugt sind die oben genannten Verbindungen der Formel **1**, wobei R¹, R² und R³ die obigen Definitionen besitzen und wobei
- R⁴: ein Rest ausgewählt aus der Gruppe bestehend aus Cl, F, Br, Methyl, Ethyl, Propyl, 2-Methylpropyl, Cyclopropyl, Cyclohexyl, Methoxy, CF₃, NR^{4.1}R^{4.2},
C₆₋₁₀-Aryl, gegebenenfalls substituiert mit einem oder mehreren Resten ausgewählt aus der Gruppe bestehend aus Methyl, CF₃, CN, Methoxy, Fluor, Chlor,
ein fünf oder sechsgliedriger heterocyclischer Ring, der ein oder mehrere Heteroatome ausgewählt aus der Gruppe bestehend aus Stickstoff und Sauerstoff enthalten kann,
ein fünfgliedriger heterocyclischer Aromat, der ein oder mehrere Reste ausgewählt aus der Gruppe bestehend aus Stickstoff und Sauerstoff enthalten kann,
ein aromatischer oder nichtaromatischer Bicyclus, der ein oder mehrere Heteroatome ausgewählt aus der Gruppe bestehend aus Schwefel und Sauerstoff enthalten kann;
R^{4.1} H, Methyl, Ethyl;
R^{4.2} Methyl, Ethyl, Phenyl; bedeuten, sowie pharmakologisch verträgliche Salze, Diastereomere, Enantiomere, Racemate, Hydrate oder Solvate davon.

Besonders bevorzugt sind die oben genannten Verbindungen der Formel 1, wobei R¹, R² und R³ die obigen Definitionen besitzen und wobei
- R⁴: Cl bedeutet,
sowie pharmakologisch verträgliche Salze, Diastereomere, Enantiomere, Racemate, Hydrate oder Solvate davon.

Ein weiterer bevorzugter Gegenstand der Erfindung sind die obigen Verbindungen der Formel **1**, wobei R¹, R² und R⁴ die obigen Definitionen besitzen und wobei
- R³: eine Gruppe der allgemeinen Formel **1a**, worin
A ein fünf-, sechs- oder siebengliedriger heterocyclischer, aromatischer oder nichtaromatischer Ring, oder ein aromatischer oder nichtaromatischer, bicyclischer Ring bestehend aus acht, neun oder zehn Atomen, der gegebenenfalls ein, zwei oder drei Heteroatome enthält;
X NR^{3.1}, O, S;
Y C₁₋₄-Alkylen, das gegebenenfalls mit einem oder mehreren R^{3.3} substituiert sein kann
m 0, 1, 2 oder 3;
R^{3.1} jeweils unabhängig voneinander C₁₋₄-Alkyl, C₆₋₁₀-Aryl, COOR^{3.1.1}, CONR^{3.1.1}R^{3.1.2}, CN, NR^{3.1.1}R^{3.1.2}, NHCOR^{3.1.1}, OR^{3.1.1}, O-C₁₋₄-Haloalkyl, SO₂R^{3.1.1}, SO₂NH₂, Halogen;
R^{3.1.1} H, C₁₋₆-Alkyl;
R^{3.1.2} H, C₁₋₆-Alkyl;
R³² H, C₁₋₆-Alkyl;
R^{3.3} jeweils unabhängig voneinander C₁₋₆-Alkyl, C₁₋₆-Alkyl-OH, C₃₋₆-Cycloalkyl, O-C₁₋₆-Alkyl, COOH, COO-C₁₋₆-Alkyl, CONH₂;
R^{3.3} bildet gemeinsam mit einem oder zwei Kohlenstoffatomen von Y einen carbocyclischen Ring mit 3, 5 oder 6 Kohlenstoffatomen
bedeutet, sowie pharmakologisch verträgliche Salze, Diastereomere, Enantiomere, Racemate, Hydrate oder Solvate davon.

Ein weiterer bevorzugter Gegenstand der Erfindung sind die obigen Verbindungen der Formel 1, wobei R¹, R² und R⁴ die obigen Definitionen besitzen und wobei
- R³: eine Gruppe der allgemeinen Formel **1a**, worin
A ein fünf-, sechs- oder siebengliedriger heterocyclischer, aromatischer oder nichtaromatischer Ring, der ein, zwei oder drei Heteroatome, unabhängig voneinander, ausgewählt aus der Gruppe bestehend aus Sauerstoff, Stickstoff und Schwefel, enthält; oder ein aromatischer oder nichtaromatischer, bicyclischer Ring bestehend aus acht, neun oder zehn Atomen, der gegebenenfalls ein, zwei oder drei Heteroatome, unabhängig voneinander, ausgewählt aus der Gruppe bestehend aus Sauerstoff, Stickstoff und Schwefel, enthält;
X NR^{3.2}, O, S;
Y C₁₋₂-Alkylen, das gegebenenfalls mit einem oder mehreren R^{3.3} substituiert sein kann
m 0, 1, 2 oder 3;
R^{3.1} jeweils unabhängig voneinander C₁₋₄-Alkyl, C₆₋₁₀-Aryl, COOH, COO-C₁₋₄-Alkyl, CONH_{2,} CN, NH₂, NHCO-C₁₋₄-Alkyl, OH, O-C₁₋₄-Alkyl, O-C₁₋₄-Haloalkyl, SO₂C₁₋₄-Alkyl, SO₂NH₂ oder Halogen;
R^{3.2} H, oder C₁₋₄-Alkyl;
R^{3.3} H, C₁₋₄-Alkyl, C₁₋₄-Alkyl-OH, C₃₋₆-Cycloalkyl, O-C₁₋₄-Alkyl, COOH, COO-C₁₋₄-Alkyl oder CONH₂
bedeutet, oder wobei
R^{3.3} gemeinsam mit einem oder zwei Kohlenstoffatomen von Y einen carbocyclischen Ring mit 3, 5 oder 6 Kohlenstoffatomen bildet, sowie pharmakologisch verträgliche Salze, Diastereomere, Enantiomere, Racemate, Hydrate oder Solvate davon.

Ein weiterer bevorzugter Gegenstand der Erfindung sind die obigen Verbindungen der Formel 1, wobei R¹, R² und R⁴ die obigen Definitionen besitzen und wobei
- R^{3.1}: jeweils unabhängig voneinander Methyl, Ethyl, Propyl, Ph, COOH, COOMe, CONH₂, CN, NH₂, NHCOMe, OH, OMe, OEt, OCF₃, OCHF₂, SO₂Me, SO₂NH₂, F, Cl, Br;
- R^{3.2}: H, C₁₋₄-Alkyl;
- R^{3.3}: H, Methyl, Ethyl, Propyl, Butyl, CH₂OH, CH₂CH₂OH, C(CH₂)₂OH, Cyclopropyl, COOH, COOMe, COOEt, COOPr, CONH₂, OMe, OEt, OPr
bedeutet oder wobei
- R^{3.3}: gemeinsam mit einem oder zwei Kohlenstoffatomen von Y einen carbocyclischen Ring mit 3, 5 oder 6 Kohlenstoffatomen bildet, sowie pharmakologisch verträgliche Salze, Diastereomere, Enantiomere, Racemate, Hydrate oder Solvate davon.

Ein weiterer bevorzugter Gegenstand der Erfindung sind die obigen Verbindungen der Formel 1, wobei R¹, R², R³ und R⁴ die obigen Definitionen besitzen und wobei
- R^{3.1}: Methyl, *iso*-Propyl, *tert*-Butyl;
- R^{3.2}: H, Methyl;
- R^{3.3}: H, Methyl;
bedeutet, sowie pharmakologisch verträgliche Salze, Diastereomere, Enantiomere, Racemate, Hydrate oder Solvate davon.

Besonders bevorzugt sind weiterhin Verbindungen der Formel **1**, worin
- R³: eine Gruppe der allgemeinen Formel **1a**, worin
A ein gesättigter oder ungesättigter, mono- oder bicyclischer C₅₋₁₀-Heterocyclus mit 1, 2 oder 3 Heteroatomen ausgewählt aus der Gruppe bestehend aus N, O und S;
X NR^{3.2}, O;
Y C₁₋₂-Alkylen, gegebenenfalls substituiert mit einem oder mehreren R^{3.3}
m 0,1, 2 oder 3;
R^{3.1} Methyl,
R^{3.2} H,
R^{3.3} H, bedeuten, sowie pharmakologisch verträgliche Salze, Diastereomere, Enantiomere, Racemate, Hydrate oder Solvate davon.

Ein weiterer bevorzugter Gegenstand der Erfindung sind die obigen Verbindungen der Formel **1**, wobei R¹, R² und R⁴ die obigen Definitionen besitzen und wobei
- R³: eine Gruppe der allgemeinen Formel **1a**, worin
A Thiophen, Furan, Pyrazol, Pyridin, Isoxazol, Thiazol, Benzimidazol, Benzo[b]thiophen, 2,3-Dihydrobenzo[1,4]dioxin, 1,2,3,4-Tetrahydronaphthalin, Oxazol, Tetrahydrofuran oder Tetrahydropyran;
X NR^{3.2}, O;
Y C₁₋₂-Alkylen, gegebenenfalls substituiert mit einem oder mehreren R^{3.3}
m 0,1, 2 oder 3;
R^{3.1} Methyl,
R^{3.2} H,
R^{3.3} H,
bedeutet, sowie pharmakologisch verträgliche Salze, Diastereomere, Enantiomere, Racemate, Hydrate oder Solvate davon.

Ein weiterer bevorzugter Gegenstand sind Verbindungen der Formel **1**, worin
- R¹: Azetidin
- R²: Piperazin
- R³: eine Gruppe der allgemeinen Formel **1a**, worin
A Thiophen, Furan, Pyrazol, Pyridin, Isoxazol, Thiazol, Benzimidazol, Benzo[b]thiophen, 2,3-Dihydrobenzo[1,4]dioxin, 1,2,3,4- Tetrahydronaphthalin, Oxazol oder Phenyl, Tetrahydrofuran oder Tetrahydropyran;
X NR^{3.2}, O;
Y methylen oder ethylen
m 0 ,1, 2 oder 3;
R^{3.1} jeweils unabhängig voneinander Methyl, Ethyl, Phenyl, Halogen, COOR^{3.1.1}, CONR^{3.1.1}R^{3.1.2}, CN, NR^{3.1.1}R^{3.1.2}, NHCOR^{3.1.1}, OR^{3.1.1}, O-C₁₋₃-Haloalkyl, SO₂R^{3.1.1}, SO₂NH₂, C₁₋₃-Haloalkyl, wobei R^{3.1.1} und R^{3.1.2} unabhängig voneinander H, Methyl, Ethyl, Propyl sein können
R^{3.2} H oder Methyl bedeuten, sowie pharmakologisch verträgliche Salze, Diastereomere, Enantiomere, Racemate, Hydrate oder Solvate davon.

Besonders bevorzugt sind Verbindungen der Formel **1** worin
- R¹: Azetidin,
- R²: Piperazin,
- R³: O-C₁₋₂-alkylen-phenyl oder NH-C₁₋₂-alkylen-phenyl
und
- R⁴: Cl bedeutet,
sowie pharmakologisch verträgliche Salze, Diastereomere, Enantiomere, Racemate, Hydrate oder Solvate davon.

Bevorzugt sind Verbindungen der Formel **1**, worin R², R³ und R⁴ die obigen Definitionen haben und worin R¹ Azetidin bedeutet, sowie pharmakologisch verträgliche Salze, Diastereomere, Enantiomere, Racemate, Hydrate oder Solvate davon.

Bevorzugt sind Verbindungen der Formel **1**, worin R¹, R³ und R⁴ die obigen Definitionen haben und worin R² Piperazin bedeutet, sowie pharmakologisch verträgliche Salze, Diastereomere, Enantiomere, Racemate, Hydrate oder Solvate davon.

Bevorzugt sind weiterhin Verbindungen der Formel **1**, worin R¹, R² und R³ die obigen Definitionen haben und worin R⁴ Cl bedeutet, sowie phannakologisch verträgliche Salze, Diastereomere, Enantiomere, Racemate, Hydrate oder Solvate davon.

Besonders bevorzugt sind die erfindungsgemäßen Verbindungen der Formel **1**:

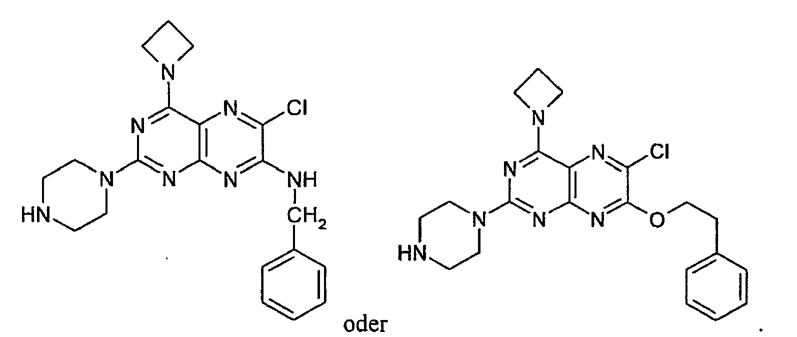

Bevorzugt sind weiterhin Verbindungen der Formel **2** 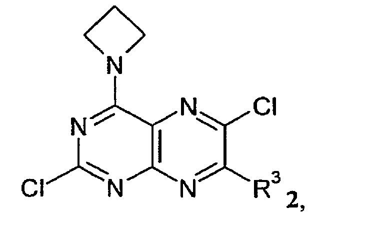 worin
- R³: eine Gruppe der Formel **1a**, 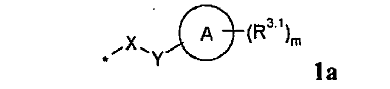 worin
A ein Ring ausgewählt aus der Gruppe bestehend aus einem monocyclischen, heterocyclischen Ring, einem bicyclischen Ring, der gegebenenfalls ein oder mehrere Heteroatome enthält, einem C₆₋₁₀-Aryl und einem C₅₋₁₀-Heteroaryl
X NR^{3.2}, O, S;
Y C₁₋₄-Alkylen, der gegebenenfalls mit einem oder mehreren R^{3.3} substituiert sein kann,
m 0, 1, 2 oder 3
R^{3.1} jeweils unabhängig voneinander C₁₋₆-Alkyl, C₆₋₁₀-Aryl, COOR^{3.1.1}, CONR^{3.1.1}R^{3.1.2}, CN, NR^{3.1.1}R^{3.1.2}, NHCOR^{3.1.1}, OR^{3.1.1}, O-C₁₋₆-Haloalkyl, SO₂R^{3.1.1}, SO₂NH₂, Halogen, C₁₋₆-Haloalkyl, C₁₋₆-alkyl-CONR^{3.1.1}R^{3.1.2}, C₁₋₆-alk^{y}l-NR^{3.1.1}R^{3.1.2}, C₁₋₆-alkyl-CONH₂, O-C₁₋₆-alkylen-NH₂, O-C₃₋₆-Cycloalkyl, O-C₁₋₄-Alkylen-C₃₋₆-cycloalkyl,O-C₁₋₄-Alkylen-CONH2, SO₂NR^{3.1.1}R^{3.1.2};
oder
- R^{3.1}: gemeinsam mit zwei Atomen von A einen 5 oder 6-gliedrigen carbocyclischen Ring oder einen 5 oder 6-gliedrigen heterocyclischen Ring bildet, der gegebenenfalls ein oder mehrere Heteroatome ausgewählt aus der Gruppe bestehend aus Sauerstoff und Stickstoff enthalten kann,
worin
R^{3.1.1} H, C₁₋₆-Alkyl;
R^{3.1.2} H, C₁₋₆-Alkyl; und
- R^{3.2}: H, C₁₋₆-Alkyl;
und worin
- R^{3.3}: jeweils unabhängig voneinander H, C₁₋₆-Alkyl, C₁₋₆-Alkyl-OH, C₃₋₆-Cycloalkyl, C₃₋₆-Cycloalkyl-OH, O-C₁₋₆-Alkyl, COOR^{3.1.1}, COO-C₁₋₆-Alkyl oder CONR^{3.1.1}R^{3.1.2} bedeuten
oder orin
- R^{3.3}: gemeinsam mit einem oder zwei Kohlenstoffatomen von Y einen carbocyclischen Ring mit 3, 4, 5 oder 6 Kohlenstoffatomen bildet,
sowie deren pharmakologisch verträgliche Salze, Diastereomere, Enantiomere, Racemate, Hydrate oder Solvate davon.

Ein weiterer Gegenstand der Erfindung sind die oben genannten Verbindungen nach Formel **1** zur Verwendung als Arzneimittel bzw. die Verwendung der oben genannten Verbindungen nach Formel 1 zur Herstellung eines Arzneimittels.

Insbesondere betrifft die vorliegende Erfindung die Verwendung der oben genannten Verbindungen nach Formel 1 zur Herstellung eines Medikaments zur Behandlung von Atemwegs- oder gastrointestinalen Beschwerden oder Erkrankungen, wie auch entzündliche Erkrankungen der Gelenke, der Haut oder der Augen, Krebserkrankungen, sowie von Erkrankungen des periphären oder zentralen Nervensystems.

Die oben genannten Verbindungen eignen sich insbesondere zur Herstellung eines Medikaments zur Vorbeugung und Behandlung von Atemwegs- oder Lungenerkrankungen, die mit einer erhöhten Schleimproduktion, Entzündungen und / oder obstruktiven Erkrankungen der Atemwege einhergehen. Hierzu gehören insbesondere die Erkrankungen COPD, chronischer Sinusitis, Asthma.

Die oben genannten Verbindungen eignen sich ebenfalls zur Herstellung eines Medikaments zur Behandlung von entzündlichen Erkrankungen des Gastrointestinaltraktes, wie z.B. Morbus Crohn, Colitis ulcerosa.

Die oben genannten Verbindungen eignen sich ebenfalls zur Herstellung eines Medikaments zur Vorbeugung und/oder zur Behandlung von Erkrankungen des periphären oder zentralen Nervensystems wie insbesondere Depression, bipolare oder manische Depression, akute und chronische Angstzustände, Schizophrenie, Alzheimer'sche Erkrankung, Parkinson'sche Erkrankung, akute und chronische Multiple Sklerose oder akute und chronische Schmerzzustände, sowie Verletzungen des Gehirns hervorgerufen durch Schlaganfall, Hypoxie oder Schädel-Hirn-Trauma.

Ebenfalls bevorzugt ist die Verwendung der oben definierten Verbindungen zur Herstellung eines Medikaments zur Behandlung von Krebserkrankungen wie z.B, akute und chronische Leukämien, akute lymphatische Leukämie (ALL) und akute myeloische Leukämie (AML), chronisch lymphatische Leukämie (CLL) und chronisch myeloische Leukämie (CML), akute nicht lymphozytische Leukämie (ANLL), Haar-Zell-Leukämie, akute promyelocytische Leukämie (APL), insbesondere die APL-Subform mit einer chromosomalen t(15; 17)-Translokation, Erkrankungen der lymphatischen Organe, Hodgkin-Lymphome und non-Hodgkin Lymphome sowie von Knochentumoren wie z.B. das Osteosarkom und sowie alle Arten von Gliomen wie z.B. Oligodendrogliom und Glioblastom.

Bei den obigen Verwendungen der erfindungsgemäßen Pteridin-Verbindungen zur Herstellung eines Medikaments zur Vorbeugung und/oder Behandlung der oben genannten Erkrankungen sind in der Regel die Nebenwirkungen der Behandlung reduziert im Vergleich zu bekannten Therapeutika nach dem Stand der Technik.

Insbesondere sind die häufig auftretenden unerwünschten Nebenwirkungen Emesis und Nausea bei der Verwendung der oben definierten Verbindungen nach Formel 1 reduziert.

### VERWENDETE BEGRIFFE UND DEFINITIONEN

Im Rahmen dieser Anmeldung können bei der Definition von möglichen Substituenten, diese auch in Form einer Strukturformel dargestellt werden. Dabei wird ein Stern (*) in der Strukturformel des Substituenten als der Verknüpfungspunkt zum Rest des Moleküls verstanden. So werden zum Beispiel die Reste N-Piperidinyl (**I**), 4-Piperidinyl (**II**), 2-Tolyl (**III**), 3-Tolyl (**IV**) und 4-Tolyl (**V**) wie folgt dargestellt:

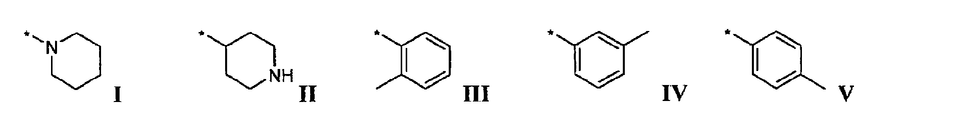

Befindet sich in der Strukturformel des Substituenten kein Stern (*), so kann an dem Substituenten jedes Wasserstoffatom entfernt werden und die dadurch freiwerdende Valenz als Bindungsstelle zum Rest eines Molekül dienen. So kann zum Beispiel **VI** die Bedeutung von 2-Tolyl, 3-Tolyl, 4-Tolyl und Benzyl haben.

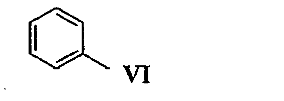

Unter pharmakologisch verträglichen Säureadditionssalzen werden beispielsweise diejenigen Salze verstanden, die ausgewählt sind aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat.

Unter dem Begriff "C₁₋₆-Alkyl" (auch soweit sie Bestandteil anderer Reste sind) werden verzweigte und unverzweigte Alkylgruppen mit 1 bis 6 Kohlenstoffatomen verstanden und unter dem Begriff "C₁₋₄-Alkyl" verzweigte und unverzweigte Alkylgruppen mit 1 bis 4 Kohlenstoffatomen verstanden. Bevorzugt sind Alkylgruppen mit 1 bis 4 Kohlenstoffatome. Beispielsweise werden hierfür genannt: Methyl, Ethyl, *n*-Propyl, *iso*-Propyl, *n*-Butyl, *iso-*Butyl, *sec*-Butyl, *tert*-Butyl, *n*-Pentyl, *iso*-Pentyl, *neo*-Pentyl oder Hexyl. Gegebenenfalls werden für vorstehend genannten Gruppen auch die Abkürzungen Me, Et, *n*-Pr, *i*-Pr, *n*-Bu, *i-*Bu, t-Bu, etc. verwendet. Sofern nicht anders beschrieben, umfassen die Definitionen Propyl, Butyl, Pentyl und Hexyl alle denkbaren isomeren Formen der jeweiligen Reste. So umfasst beispielsweise Propyl *n*-Propyl und *iso*-Propyl, Butyl umfasst *iso*-Butyl, *sec*-Butyl und *tert-*Butyl etc.

Unter dem Begriff "C₁₋₆-Alkanol" (auch soweit sie Bestandteil anderer Reste sind) werden verzweigte und unverzweigte Alkylgruppen mit 1 bis 6 Kohlenstoffatomen, die mit einem oder mehreren Hydroxylgruppen substituiert sind, verstanden und unter dem Begriff "C₁₋₄-Alkanol" werden verzweigte und unverzweigte Alkylgruppen mit 1 bis 4 Kohlenstoffatomen verstanden, die mit einer oder mehreren Hydroxylgruppen substituiert sind. C₁₋₆-Alkanole, die mit einem Hydroxylgruppen substituiert sind, werden auch als "einwertige" C₁₋₆-Alkanole bezeichnet. C₁₋₆-Alkanole, die mit zwei oder mehreren Hydroxylgruppen substituiert sind, werden auch als "mehrwertige" C₁₋₆-Alkanole bezeichnet. Bevorzugt sind Alkanolgruppen mit 1 bis 4 Kohlenstoffatome. Beispielsweise werden hierfür genannt: CH₂-OH, Ethyl-OH, *n*-Propyl-OH, *n*-Butyl-OH, *iso*-Propyl-OH, *n*-Butyl-OH, *iso-*Butyl-OH, *sec*-Butyl-OH, *tert*-Butyl-OH, *n*-Pentyl-OH, *iso*-Pentyl-OH, *neo*-Pentyl-OH,

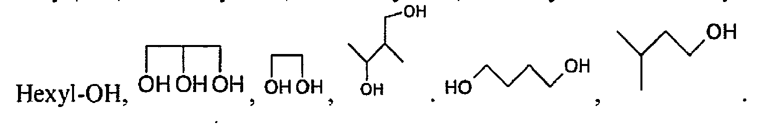

Unter dem Begriff "C₁₋₄-Alkylen" (auch soweit sie Bestandteil anderer Reste sind) werden verzweigte und unverzweigte Alkylengruppen mit 1 bis 4 Kohlenstoffatomen verstanden. Beispielsweise werden hierfür genannt: Methylen, Ethylen, Propylen, 1-Methylethylen, Butylen, 1-Methylpropylen, 1,1-Dimethylethylen oder 1,2-Dimethylethylen. Sofern nicht anders beschrieben, umfassen die Definitionen Propylen und Butylen alle denkbaren isomeren Formen der jeweiligen Reste gleicher Kohlenstoffanzahl. So umfasst beispielsweise Propyl auch 1-Methylethylen und Butylen umfasst 1-Methylpropylen, 1,1-Dimethylethylen, 1,2-Dimethylethylen.

Unter dem Begriff "C₃₋₆-Cycloalkyl" (auch soweit sie Bestandteil anderer Reste sind) werden cyclische Alkylgruppen mit 3 bis 6 Kohlenstoffatomen verstanden. Beispielsweise werden hierfür genannt: Cyclopropyl, Cyclobutyl, Cyclopentyl oder Cyclohexyl

Unter dem Begriff " C₇₋₁₁-Aralkyl" (auch soweit sie Bestandteil anderer Reste sind) werden verzweigte und unverzweigte Alkylgruppen mit 1 bis 5 Kohlenstoffatomen verstanden, die mit einem aromatische Ringsystem mit 6 Kohlenstoffatomen substituiert sind. Beispielsweise werden hierfür genannt: Benzyl, 1- oder 2-Phenylethyl. Halogen steht im Rahmen der vorliegenden Erfindung für Fluor, Chlor, Brom oder Jod. Sofern nicht gegenteilig angegeben, gelten Fluor, Chlor und Brom als bevorzugte Halogene.

Unter dem Begriff "C₁₋₆-Haloalkyl" (auch soweit sie Bestandteil anderer Reste sind) werden verzweigte und unverzweigte Alkylgruppen mit 1 bis 6 Kohlenstoffatomen verstanden, die mit einem oder mehreren Halogenatomen substituiert sind. Unter dem Begriff "C₁₋₄-Alkyl" verzweigte und unverzweigte Alkylgruppen mit 1 bis 4 Kohlenstoffatomen verstanden, die mit einem oder mehreren Halogenatomen substituiert sind. Bevorzugt sind Alkylgruppen mit 1 bis 4 Kohlenstoffatome. Beispielsweise werden hierfür genannt: CF₃, CHF₂, CH₂F, CH₂CF₃.

Unter dem Begriff "Aryl" oder "C₆₋₁₀-Aryl" (auch soweit sie Bestandteil anderer Reste sind) werden aromatische Ringsysteme mit 6 oder 10 Kohlenstoffatomen verstanden. Beispielsweise werden hierfür genannt: Phenyl oder Naphthyl, bevorzugter Arylrest ist Phenyl.

Unter dem Begriff "heterocyclische Ringe", "Heterocyclus" oder "Het" werden fünf-, sechs- oder siebengliedrige, gesättigte oder ungesättigte heterocyclische Ringe oder 5-10 gliedrige, bicyclische Heteroringe verstanden die ein, zwei oder drei Heteroatome, ausgewählt aus der Gruppe Sauerstoff, Schwefel und Stickstoff enthalten können, dabei kann der Ring über ein Kohlenstoffatom oder falls vorhanden über ein Stickstoffatom mit dem Molekül verknüpft sein. Als Beispiele für fünf-, sechs- oder siebengliedrige, gesättigte oder ungesättigte heterocyclische Ringe, werden genannt:

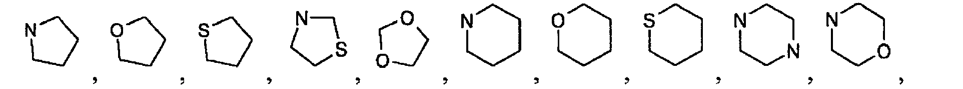

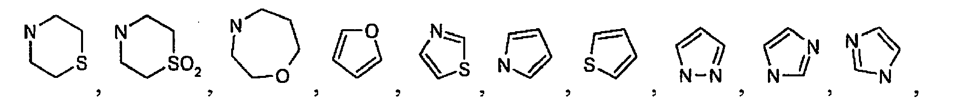

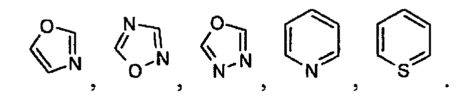

Soweit nicht anders erwähnt, kann ein heterocyclischer Ring mit einer Ketogruppe versehen sein. Als Beispiel hierfür werden genannt.

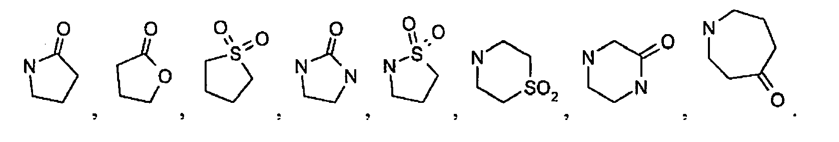

Als Beispiel für sieben-, acht-, neun-, oder zehngliedrige gesättigte, ungesättigte oder teilweise ungesättigte bicyclische Heterocyclen werden genannt Pyrrolizin, Indol, Indolizin, Isoindol, Indazol, Purin, Chinolin, Isochinolin, Benzimidazol, Benzofuran, Benzopyran, Benzothiazol, Benzoisothiazol, Pyridopyrimidin, Pteridin, Pyrimidopyrimidin,

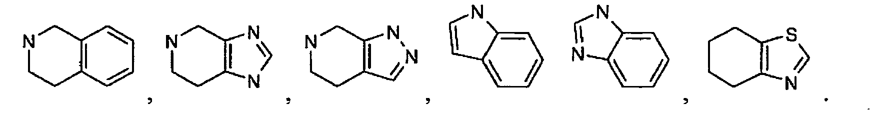

Obwohl unter dem Begriff "heterocyclische Ringe" oder "Heterocyclus" umfasst, definiert der Begriff "Heteroaromat" oder "Heteroaryl" fünf- oder sechsgliedrige heterocyclische monocyclische Aromaten oder 5-10 gliedrige, bicyclische Heteroarylringe, die ein, zwei oder drei Heteroatome ausgewählt aus der Gruppe Sauerstoff, Schwefel und Stickstoff, enthalten können und so viele konjugierte Doppelbindungen enthalten, dass ein aromatisches System gebildet wird. Der Ring kann über ein Kohlenstoffatom oder -falls vorhanden- über ein Stickstoffatom mit dem Molekül verknüpft sein. Als Beispiele für fünf- oder sechsgliedrige Heteroaryle werden genannt:

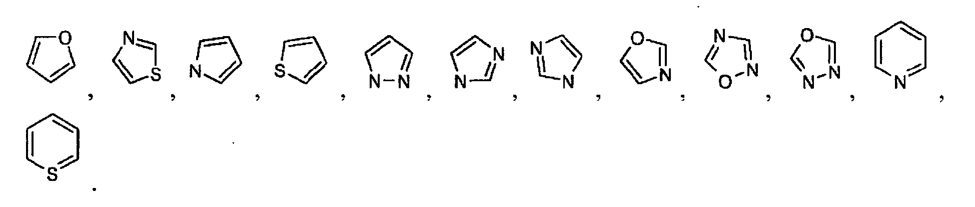

### BEISPIELE

Die erfindungsgemäßen Verbindungen können nach an sich aus der Literatur bekannten Methoden hergestellt werden, wie sie zum Beispiel in DE 3540952 beschrieben sind. Weitere Herstellvarianten für die unten gezeigten Verbindungen sind im folgenden Reaktionsschema 1 dargestellt und in den Beispielen detailliert beschrieben.

### Beispiel 1: 4-Azetidin-1-yl-7-benzylamino-6-chlor-2-piperazin-1-yl-pteridin:

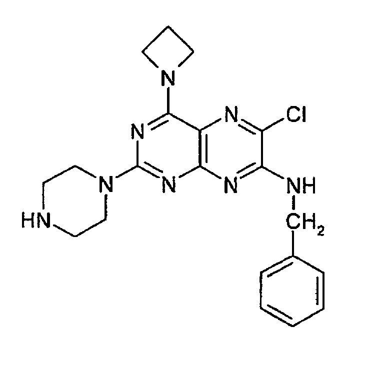

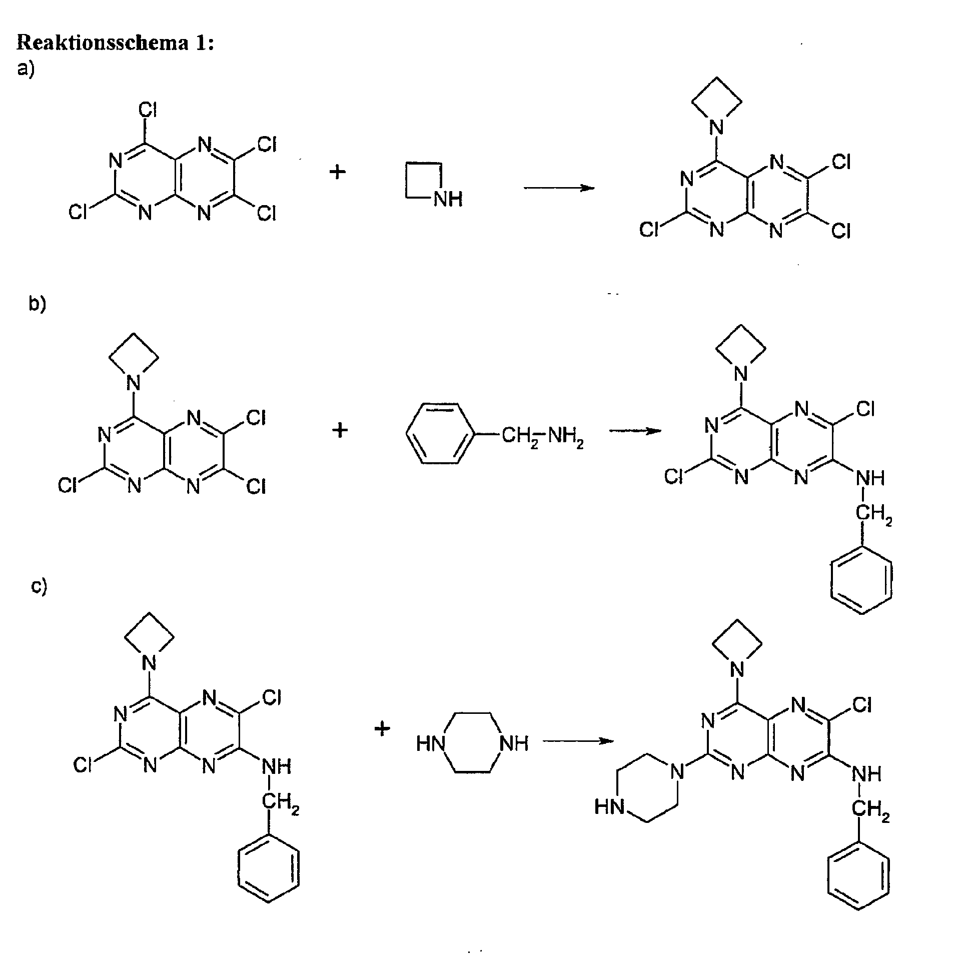

### a) 4-Azetidin-1-yl-2,6,7-trichlor-pteridin: 2 g (7,4 mmol) Tetrachlorpteridin werden in ca. 150 ml Chloroform gelöst und mit einer Lösung von 1,25 g (14 mmol)

Natriumhydrogencarbonat in 60 ml Wasser versetzt. Man kühlt auf 0° C, versetzt mit einer Lösung von 0,5 ml (7,4 mmol) Azetidin in ca. 50 ml Chloroform und rührt eine Stunde bei 0° C weiter. Dann wird die organische Phase abgetrennt, mit Wasser gewaschen, über Natriumsulfat getrocknet und im Vakuum vom Lösungsmittel befreit. Der Rückstand wird mit Ether verrieben und abgesaugt. Ausbeute 780 mg (36% d. Th.), die Substanz wird ohne weitere Reinigung im nächsten Schritt eingesetzt.

### b) 4-Azetidin-1-yl-7-benzylamino-2,6-dichlor-pteridin: 740 mg (2,5 mmol) 4-Azetidin-1-yl-2,6,7-trichlor-pteridin werden zusammen mit 0,45 ml (2,5 mmol)

Diisopropylethylamin in ca. 30 ml Dioxan gelöst. Dazu tropft man unter Rühren eine Lösung von 0,28 ml (2,5 mmol) Benzylamin in ca. 5 ml Dioxan. Nach beendeter Zugabe wird für 0,5 h weiter gerührt. Das Reaktionsgemisch wird in 35 ml Eiswasser gegossen und gerührt, dabei fällt ein gelber Niederschlag aus der abgesaugt wird. Der Niederschlag wird in Dichlormethan aufgenommen und mit Wasser gewaschen. Die organische Phase wird mit Natriumsulfat getrocknet und im Vakuum vom Lösungsmittel befreit. Der Rückstand wird chromatographisch gereinigt, Ausbeute 300 mg (32% d. Th.).

### c) 4-Azetidin-1-yl-7-benzylamino-6-chlor-2-piperazin-1-yl-pteridin:

300 mg (0,83 mol) 4-Azetidin-1-yl-7-benzylalmino-2,6-dichlor-pteridin werden in 25 ml Dioxan suspendiert und langsam zu einer auf 80° C erhitzten Lösung von 0,36 g (4,2 mmol) Piperazin in 20 ml Dioxan getropft. Nach beendeter Zugabe wird für 20 min weiter gerührt und das Reaktionsgemisch in Wasser gegossen. Man extrahiert mit Dichlormethan, die organische Phase wird über Natriumsulfat getrocknet und im Vakuum vom Lösungsmittel befreit. Der Rückstand wird chromatographisch gereinigt, Ausbeute 80 mg (23% d. Th.).M+H = 411 /413

### Beispiel 2: 4-Azetidin-1-yl-6-chlor-7-(2-phenylethyloxy)-2-piperazin-1-yl-pteridin:

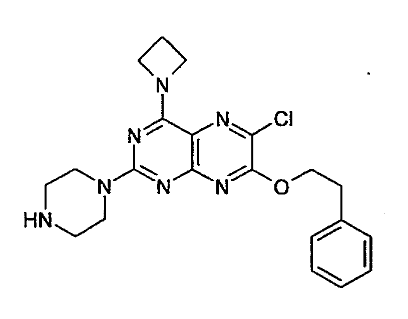

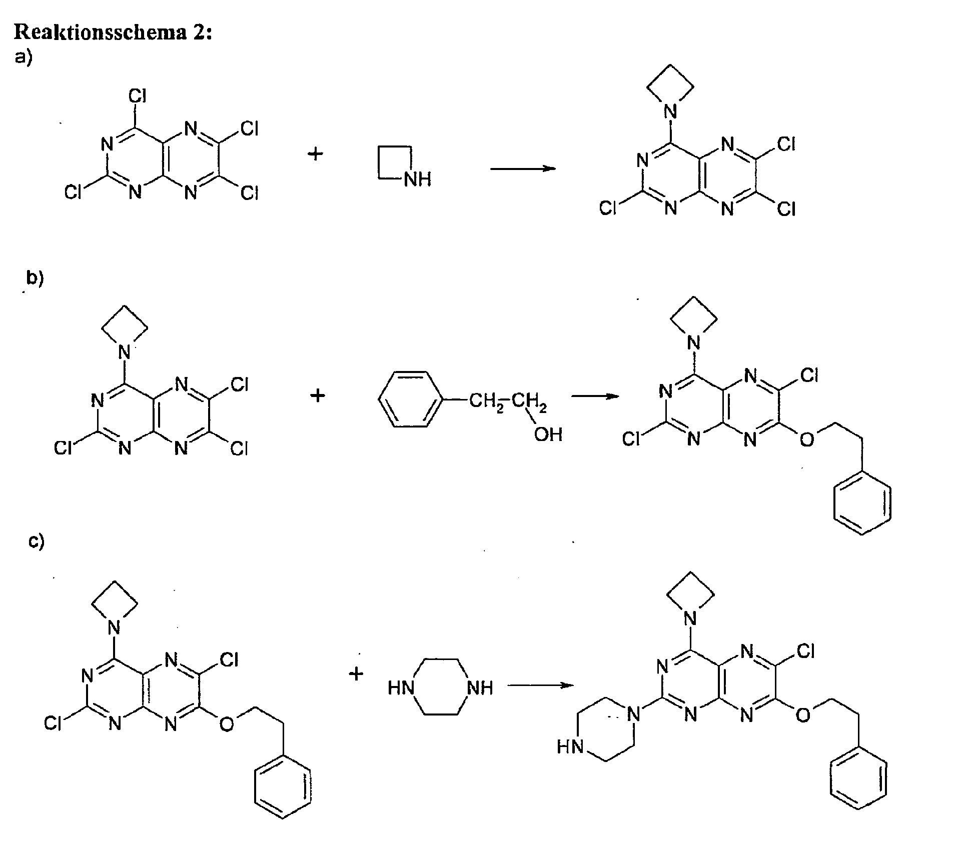

a) 4-Azetidin-1-yl-2.6.7-trichlor-pteridin wird wie für Beispiel 1 beschrieben hergestellt.
b) 4-Azetidin-1-yl-2,6,-dichlor-7-(2-phenylethloxy)-pteridin: 82 µl (0,69 mmol) 2-Phenylethanol werden in 5 ml Tetrahydrofuran gelöst. Man kühlt auf ca. -5° C und gibt unter Rühren 0,35 ml (0,7 mmol) einer 2 molaren Lösung von Lithiumdiisopropylamid in Tetrahydrofuran zu, anschließend wird 30 min bei Raumtemperatur gerührt. Man kühlt wieder auf-5° C und versetzt mit einer Lösung von 200 mg (0,69 mmol) 4-Azetidin-1-yl-2,6,7-trichlor-pteridin in 10 ml Tetrahydrofuran. Man lässt die Mischung auf Raumtemperatur kommen und rührt über Nacht weiter. Man versetzt mit ca. 50 ml Wasser und extrahiert mit Dichlormethan. Die organische Phase wird mit Wasser gewaschen, über Natriumsulfat getrocknet und im Vakuum vom Lösungsmittel befreit. Der Rückstand wird chromatographisch gereinigt. Ausbeute 111 mg (43% d. Th.).
c) 4-Azetidin-1-yl-6-chlor-7-(2-phenylethyloxyloxy)-2-piperazin-1-yl-pteridin: 115 mg (1,3 mmol) Piperazin werden in 2 ml Dioxan suspendiert und auf 80° C erhitzt. Anschließend wird eine Lösung aus 100 mg (0,27 mmol) 4-Azetidin-1-yl-2,6,-dichlor-7-(2-phenethyloxy)-pteridin in 5 ml Dioxan zugetropft, die Mischung wird für 16 h bei 80° C gerührt. Anschließend wird das Reaktionsgemisch im Vakuum vom Lösungsmittel befreit, der Rückstand wird chromatographisch gereinigt. Ausbeute 82 mg (72% d. Th.).

Die Synthese von Tetrachlorpteridin, welches das Ausgangsprodukt für die Synthesen aus Reaktionsschema 1 und 2 ist, ist beschrieben in: Schöpf, C.; Reichert, R.; Riefstahl, K. Liebigs Ann. Chem. (1941), 548, 82 - 94. M +H = 426 / 428

### INDIKATIONSGEBIETE

Wie gefunden wurde, zeichnen sich die Verbindungen der Formel 1 durch vielfältige Anwendungsmöglichkeiten auf therapeutischem Gebiet aus. Hervorzuheben sind solche Anwendungsmöglichkeiten, für welche die erfindungsgemäßen Verbindungen der Formel 1 aufgrund ihrer pharmazeutischen Wirksamkeit als PDE4-lnhibitor bevorzugt zur Anwendung gelangen können. Beispielhaft genannt seien Atemwegs- oder gastrointestinale Erkrankungen oder Beschwerden, entzündliche Erkrankungen der Gelenke, der Haut oder der Augen, Krebserkrankungen, sowie Erkrankungen des periphären oder zentralen Nervensystems.

Hierbei bevorzugt genannt sei die Vorbeugung und Behandlung von Atemwegs- oder Lungenerkrankungen, welche mit einer erhöhten Schleimproduktion, Entzündungen und/oder obstruktiven Erkrankungen der Atemwege einher gehen. Beispielhaft hierfür seien genannt, akute, allergische oder chronische Bronchitis, chronisch obstruktive Bronchitis (COPD), Husten, Lungenemphysem, allergische oder nicht-allergische Rhinitis oder Sinusitis, chronische Rhinitis oder Sinusitis, Asthma, Alveolitis, Farmers' Krankheit, hyperreaktive Atemwege, infektiöse Bronchitis oder Pneumonitis, pediatrisches Asthma, Bronchiektasien, Lungenfibrose, ARDS (akutes Atemnotsyndrom des Erwachsenen), Bronchialödem, Lungenödem, Bronchitis, Pneumonie oder interstitielle Pneumonie ausgelöst durch verschiedene Ursachen wie Aspiration, Inhalation von toxischen Gasen oder Bronchitis, Pneumonie oder interstitielle Pneumonie ausgelöst durch Herzinsuffizienz, Bestrahlung, Chemotherapie zystische Fibrose (Mukoviszidose), alpha1-Antitrypsin-Mangel.

Ebenfalls bevorzugt genannt sei die Behandlung von entzündlichen Erkrankungen des Gastrointestinaltraktes. Beispielhaft hierfür seien genannt, akute oder chronische entzündliche Veränderungen bei Gallenblasenentzündung, Morbus Crohn, Colitis ulcerosa, entzündliche Pseudopolypen, juvenile Polypen, Colitis cystica profunda, pneumatosis cystoides interstinales, Erkrankungen der Gallengänge und Gallenblase, z.B. Gallensteine und Konglomerate, zur Behandlung von entzündlichen Erkrankungen der Gelenke wie rheumatoide Arthritis oder entzündliche Erkrankungen der Haut und der Augen.

Ebenfalls bevorzugt genannt sei die Behandlung von Krebserkrankungen. Beispielhaft hierfür seien genannt alle Formen von akuten und chronischen Leukämien wie akute lymphatische Leukämie (ALL), akute myeloische Leukämie (AML), akute nicht lymphozytische Leukämie (ANLL), chronisch lymphatische Leukämie (CLL), chronisch myeloische Leukämie (CML), Haar-Zell-Leukämie, akute promyelocytische Leukämie (APL), insbesondere die APL-Subform mit einer chromosomalen t(15; 17)-Translokation, Erkrankungen der lymphatischen Organe, Hodgkin-Lymphome und non-Hodgkin Lymphome, sowie Knochentumoren wie das Osteosarkom und sowie alle Arten von Gliomen wie Oligodendrogliom und Glioblastom.

Des Weiteren bevorzugt genannt sei die Vorbeugung und Behandlung von Erkrankungen des periphären oder zentralen Nervensystems. Beispielhaft hierfür seien genannt Depression, bipolare oder manische Depression, akute und chronische Angstzustände, Schizophrenie, Alzheimer'sche Erkrankung, Parkinson'sche Erkrankung, akute und chronische Multiple Sklerose oder akute und chronische Schmerzzustände sowie Verletzungen des Gehirns hervorgerufen durch Schlaganfall, Hypoxie oder Schädel-Him-Trauma.

Besonders bevorzugt betrifft die vorliegende Erfindung die Verwendung von Verbindungen der Formel 1 zur Herstellung eines Arzneimittels zur Behandlung entzündlicher oder obstruktiver Erkrankungen der oberen und unteren Atmungsorgane einschließlich der Lunge wie beispielsweise allergische Rhinitis, chronische Rhinitis, Bronchiectasis, zystische Fibrose, idiopathische Lungenfibrose, fibrosierende Alveolitis, COPD, chronische Bronchitis, chronischer Sinusitis, Asthma, insbesondere COPD, chronische Bronchitis und Asthma.

Am meisten bevorzugt ist die Verwendung der Verbindungen der Formel 1 Zur Behandlung von entzündlichen und obstruktiven Erkrankungen wie COPD, chronische Bronchitis, chronischer Sinusitis, Asthma, Morbus Crohn, Colitis ulcerosa, insbesondere COPD, chronische Bronchitis und Asthma.

Ebenfalls bevorzugt ist die Verwendung der Verbindungen der Formel 1 zur Behandlung von Erkrankungen des periphären oder zentralen Nervensystems wie Depression, bipolare oder manische Depression, akute und chronische Angstzustände, Schizophrenie, Alzheimer'sche Erkrankung, Parkinson'sche Erkrankung, akute und chronische Multiple Sklerose oder akute und chronische Schmerzzustände sowie Verletzungen des Gehirns hervorgerufen durch Schlaganfall, Hypoxie oder Schädel-Hirn-Trauma.

Ein herausragender Aspekt der vorliegenden Erfindung ist das reduzierte Profil an Nebenwirkungen. Darunter wird im Rahmen der Erfindung verstanden, eine Dosis einer pharmazeutischen Zusammensetzung verabreichen zu können, ohne beim Patienten Erbrechen, bevorzugt Übelkeit, besonders bevorzugt Unwohlsein auszulösen. Höchst bevorzugt ist die Verabreichung einer therapeutisch wirksamen Substanzmenge, ohne Emesis oder Nausea auszulösen, in jedem Stadium des Krankheitsverlaufs.

### DARREICHUNGSFORMEN

Einen weiteren Aspekt der Erfindung bilden Medikamente zur Behandlung von Atemwegserkrankungen, die eines oder mehrere der oben genannten Pteridine der Formel 1 enthalten.

Geeignete Anwendungsformen sind beispielsweise Tabletten, Kapsel, Lösungen, Säfte, Emulsionen oder Inhalationspulver oder -aerosole. Hierbei soll der Anteil der pharmazeutisch wirksamen Verbindung(en) jeweils im Bereich von 0,1 bis 90 Gew.-%, bevorzugt 0,5 bis 50 Gew.-% der Gesamtzusammensetzung liegen, d.h. in Mengen die ausreichend sind, um den unten angegebenen Dosierungsbereich zu erreichen.

Die orale Gabe kann in Form einer Tablette, als Pulver, als Pulver in einer Kapsel (z.B. Hartgelatinekapsel), als Lösung oder Suspension erfolgen. Im Fall einer inhalativen Gabe kann die Wirkstoffkombination als Pulver, als wässrige oder wässrig-ethanolische Lösung oder mittels einer Treibgasformulierung erfolgen.

Bevorzugt sind deshalb pharmazeutische Formulierungen gekennzeichnet durch den Gehalt an einer oder mehrerer Verbindungen der Formel 1 gemäß der obigen bevorzugten Ausführungsformen.

Besonders bevorzugt ist es, wenn die Verbindungen der Formel 1 oval verabreicht werden, besonders bevorzugt ist es, wenn die Verabreichung ein oder zweimal täglich erfolgt. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein Geschmack verbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie *p*-Hydroxybenzoate, enthalten.

Die eine oder mehrere Wirkstoffe beziehungsweise Wirkstoffkombinationen enthaltenden Kapseln können beispielsweise hergestellt werden, indem man die Wirkstoffe mit inerten Trägem, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuss- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mit verwendet werden. Im Falle wässriger Suspensionen können die Wirkstoffe außer den oben genannten Hilfsstoffen mit verschiedenen Geschmack-Aufbesserern oder Farbstoffen versetzt werden.

Ebenfalls bevorzugt ist es, wenn die Verbindungen der Formel **1** inhalativ verabreicht werden, besonders bevorzugt ist es, wenn die Verabreichung ein oder zweimal täglich erfolgt. Hierzu müssen die Verbindungen der Formel **1** in inhalierbaren Darreichungsformen bereitgestellt werden. Als inhalierbare Darreichungsformen kommen Inhalationspulver, treibgashaltige Dosieraerosole oder treibgasfreie Inhalationslösungen in Betracht, die gegebenenfalls im Gemisch mit gebräuchlichen physiologisch verträglichen Hilfsstoffen vorliegen.

Im Rahmen der vorliegenden Erfindung sind von dem Begriff treibgasfreie Inhaltionslösungen auch Konzentrate oder sterile, gebrauchsfertige Inhalationslösungen umfasst. Die im Rahmen der vorliegenden Erfindung einsetzbaren Darreichungsformen werden im nachfolgenden Teil der Beschreibung detailliert beschrieben.

### Inhalationspulver

Sind die Verbindungen der Formel **1** im Gemisch mit physiologisch unbedenklichen Hilfsstoffen enthalten, können zur Darstellung der erfindungsgemäßen Inhalationspulver die folgenden physiologisch unbedenklichen Hilfsstoffe zur Anwendung gelangen: Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung. Verfahren zur Herstellung der erfindungsgemäßen Inhalationspulver durch Mahlen und Mikronisieren sowie durch abschließendes Mischen der Bestandteile sind aus dem Stand der Technik bekannt.

### Treibgashaltige Inhalationsaerosole

Die im Rahmen der erfindungsgemäßen Verwendung einsetzbaren treibgashaltigen Inhalationsaerosole können 1 im Treibgas gelöst oder in dispergierter Form enthalten. Die zur Herstellung der Inhalationsaerosole einsetzbaren Treibgase sind aus dem Stand der Technik bekannt. Geeignete Treibgase sind ausgewählt aus der Gruppe bestehend aus Kohlenwasserstoffen wie n-Propan, n-Butan oder Isobutan und Halogenkohlenwasserstoffen wie bevorzugt fluorierten Derivaten des Methans, Ethans, Propans, Butans, Cyclopropans oder Cyclobutans. Die vorstehend genannten Treibgase können dabei allein oder in Mischungen derselben zur Verwendung kommen. Besonders bevorzugte Treibgase sind fluorierte Alkanderivate ausgewählt aus TG134a (1,1,1,2-Tetrafluorethan), TG227 (1,1,1,2,3,3,3-Heptafluorpropan) und Mischungen derselben. Die im Rahmen der erfindungsgemäßen Verwendung einsetzbaren treibgashaltigen Inhalationsaerosole können ferner weitere Bestandteile wie Co-Solventien, Stabilisatoren, oberflächenaktive Mittel (surfactants), Antioxidantien, Schmiermittel sowie Mittel zur Einstellung des pH-Werts enthalten. All diese Bestandteile sind im Stand der Technik bekannt.

### Treibgasfreie Inhalationslösungen

Die erfindungsgemäße Verwendung von Verbindungen der Formel 1 erfolgt bevorzugt zur Herstellung von treibgasfreien Inhalationslösungen und Inhalationssuspensionen. Als Lösungsmittel kommen hierzu wässrige oder alkoholische, bevorzugt ethanolische Lösungen in Betracht. Das Lösungsmittel kann ausschließlich Wasser sein oder es ist ein Gemisch aus Wasser und Ethanol. Die Lösungen oder Suspensionen werden mit geeigneten Säuren auf einen pH-Wert von 2 bis 7, bevorzugt von 2 bis 5 eingestellt. Zur Einstellung dieses pH-Werts können Säuren ausgewählt aus anorganischen oder organischen Säuren Verwendung finden. Beispiele für besonders geeignete anorganische Säuren sind Salzsäure, Brom wasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure, Schwefelsäure. Es können auch die Säuren verwendet werden, die bereits mit einem der Wirkstoffe ein Säureadditionssalz bilden. Unter den organischen Säuren sind Ascorbinsäure, Fumarsäure und Zitronensäure bevorzugt. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe, Antioxidantien oder Komplexbildner besitzen, wie beispielsweise Zitronensäure oder Ascorbinsäure. Erfindungsgemäß besonders bevorzugt wird Salzsäure zur Einstellung des pH-Werts verwendet.

Den im Rahmen der erfindungsgemäßen Verwendung einsetzbaren treibgasfteien Inhalationslösungen können Co-Solventien und/oder weitere Hilfsstoffe zugesetzt werden. Bevorzugte Co-Solventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester.

Unter Hilfs- und Zusatzstoffen wird in diesem Zusammenhang jeder pharmakologisch verträgliche Stoff verstanden, der kein Wirkstoff ist, aber zusammen mit dem (den) Wirkstoff(en) in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe, wie z.B. Sojalecithin, Ölsäure, Sorbitanester, wie Polysorbate, Polyvinylpyrrolidon sonstige Stabilisatoren, Komplexbildner, Antioxidantien und/oder Konservierungsstoffe, die die Verwendungsdauer der fertigen Arzneimittelformulierung gewährleisten oder verlängern, Geschmackstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid als Isotonantien. Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine. Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniumchlorid, Benzalkoniumchlorid oder Benzoesäure bzw. Benzoate wie Natriumbenzoat in der aus dem Stand der Technik bekannten Konzentration.

Für die oben beschriebenen Behandlungsformen werden gebrauchsfertige Packungen eines Medikaments zur Behandlung von Atemwegserkrankungen, beinhaltend eine beigelegte Beschreibung, welche beispielsweise die Worte Atemwegserkrankung, COPD oder Asthma enthalten, und ein Pteridin der Formel 1 bereit gestellt.

## Patentansprüche

1. Verbindungen der Formel 1, 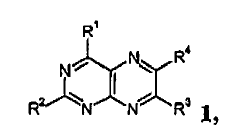 worin
R¹ ein gesättigter oder ungesättigter, viergliedriger Heterocyclus, der ein Stickstoffatom enthält und gegebenenfalls ein weiteres Atom ausgewählt aus der Gruppe bestehend aus Stickstoff, Schwefel und Sauerstoff enthalten kann;
R² Halogen, OR^{2.1}, SR^{2.1}, NR^{2.1}R^{2.2},
wobei
R^{2.1} H, C₁₋₄-Alkyl, C₆₋₁₀-Aryl, C₇₋₁₁- Aralkyl;
R^{2.2} H, C₁₋₄-Alkyl, C₆₋₁₀-Aryl, C₇₋₁₁- Aralkyl;
oder
R² ein Rest ausgewählt aus der Gruppe bestehend aus C₆₋₁₀-Aryl, C₅₋₁₀-Heteroarylund einem fünf-, sechs- oder siebengliedrigen Heterocyclus, der ein Stickstoffatom enthält und der Gegebenenfalls ein weiteres Atom ausgewählt aus der Gruppe bestehend aus Stickstoff, Schwefel und Sauerstoff enthalten kann, wobei dieser Rest gegebenenfalls mit einem Rest ausgewählt aus der Gruppe bestehend aus C₁₋₆-Alkyl, O-C₁₋₆-Alkyl, C₃₋₆-Cycloalkyl, C₇₋₁₁- Aralkyl und N(C₁₋₄-Alkyl)₂ substituiert sein kann
R³ eine Gruppe der Formel **1a**, 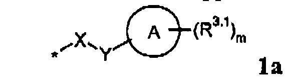 worin
A ein Ring ausgewählt aus der Gruppe bestehend aus einem monocyclischen, heterocyclischen Ring, einem bicyclischen Ring, der gegebenenfalls ein oder mehrere Heteroatome enthält, einem C₆₋₁₀-Aryl und einem C₅₋₁₀-Heteroaryl
X NR^{3.2}, O,S;
Y C₁₋₄-Alkylen, der gegebenenfalls mit einem oder mehreren R^{3.3} substituiert sein kann,
m 0, 1, 2 oder 3
R^{3.1} jeweils unabhängig voneinander C₁₋₆-Alkyl, C₆₋₁₀-Aryl, COOR^{3.1.1}, CONR^{3.1.1}R^{3.1.2}, CN, NR^{3.1.1}R^{3.1.2}, NHCOR^{3.1.1}, OR^{3.1.1}, O-C₁₋₆-Haloalkyl, SO₂R^{3.1.1}, SO₂NH₂, Halogen, C₁₋₆-Haloalkyl, C₁₋₆-alkyl-CONR^{3.1.1}R^{3.1.2}, C₁₋₆-alkyl-NR^{3.1.1}R^{3.1.2}, C₁₋₆-alkyl-CONH₂, O-C₁₋₆-alkylen-NH₂, O-C₃₋₆-Cycloalkyl, O-C₁₋₆-Alkylen,C₃₋₆-cycloalkyl,O-C₁₋₄-Alkylen-CONH₂, SO₂NR^{3.1.1}R^{3.1.2};
oder
R^{3.1} bildet gemeinsam mit zwei Atomen von A einen 5 oder 6-gliedrigen carbocyclischen Ring oder einen 5 oder 6-gliedrigen heterocyclischen Ring, der gegebenenfalls ein oder mehrere Heteroatome ausgewählt aus der Gruppe bestehend aus Sauerstoff und Stickstoff enthalten kann,
worin
R^{3.1.1} H, C₁₋₆-Alkyl;
R^{3.1.2} H, C₁₋₆-Alkyl;
und
R^{3.2} H, C₁₋₆-Alkyl;
und worin
R^{3.3} oder jeweils unabhängig voneinander H, C₁₋₆-Alkyl, C₁₋₆-Alkyl-OH, C₃₋₆-Cycloalkyl, C₃₋₆-Cycloalkyl-OH, O-C₁₋₆-Alkyl, COOR^{3.1.1}, COO-C₁₋₆-Alkyl, CONR^{3.1.1}R^{3.1.2} ;
R^{3.3} bildet gemeinsam mit einem oder zwei Kohlenstoffatomen von Y einen carbocyclischen Ring mit 3, 4, 5 oder 6 Kohlenstoffatomen,
R⁴ ein Rest ausgewählt aus der Gruppe bestehend aus Halogen, C₁₋₆-Alkyl, C₂₋₆-Alkenyl, C₂₋₆₋Alkinyl, C₃₋₆-Cycloalkyl, C₃₋₆-Cycloalkenyl, OR^{4.1}, SR^{4.1}, C₁₋₆-Haloalkyl, NR^{4.1}R^{4.2} oder ein Rest ausgewählt aus der Gruppe bestehend aus C₆₋₁₀-Aryl, 3-10-gliedriger Heterocyclus und C₅₋₁₀-Heteroaryl, der gegebenenfalls mit einem oder mehreren Resten ausgewählt aus der Gruppe bestehend aus C₁₋₆-Alkyl, C₁₋₆-Haloalkyl, CN, O-C₁₋₆-Alkyl, Halogen substituiert sein kann,
R^{4.1} H, C₁₋₆-Alkyl, C₆₋₁₀-Aryl, C₇₋₁₁- Aralkyl;
R^{4.2} H, C₁₋₆-Alkyl, C₆₋₁₀-Aryl, C₇₋₁₁- Aralkyl;
bedeuten, sowie pharmakologisch verträgliche Salze, Diastereomere, Enantiomere, Racemate, Hydrate oder Solvate davon.

2. Verbindungen der Formel **1,** nach Anspruch 1, worin
R¹ ein Azetidinring
bedeutet, sowie pharmakologisch verträgliche Salze, Diastereomere, Enantiomere, Racemate, Hydrate oder Solvate davon.

3. Verbindungen der Formel **1,** nach Anspruch 1 oder 2, worin
Ru 1 ein Azetidinring ist,
R² ein fünf- oder sechsgliedriger Heterocyclus ist, der ein oder zwei Stickstoffatome enthält;
bedeuten, sowie pharmakologisch verträgliche Salze, Diastereomere, Enantiomere, Racemate, Hydrate oder Solvate davon.

4. Verbindungen der Formel **1,** nach einem der Ansprüche 1 bis 3, worin
R¹ Azetidinring ist;
R² ein sechsgliedriger Heterocyclus ist, der zwei Stickstoffatome enthält,
bedeuten, sowie pharmakologisch verträgliche Salze, Diastereomere, Enantiomere, Racemate, Hydrate oder Solvate davon,

5. Verbindungen der Formel **1**, nach einem der Ansprüche 1 bis 4, worin
R⁴ ein Rest ausgewählt aus der Gruppe bestehend aus Cl, F, Br, Methyl, Methyl, Propyl, 2-Methylpropyl, Cyclopropyl, Cyclohexyl, Methoxy, CF₃, NR^{4.1}R^{4.2},
C₆₋₁₀-Aryl, gegebenenfalls substituiert mit einem oder mehreren Resten ausgewählt aus der Gruppe bestehend aus Methyl, CF₃, CN, Methoxy, Fluor, Chlor,
ein fünf oder sechsgliedriger heterocyclischer Ring, der ein oder mehrere Heteroatome ausgewählt aus der Gruppe bestehend aus Stickstoff und Sauerstoff enthalten kann,
ein fünfgliedriger heterocyclischer Aromat, der ein oder mehrere Reste ausgewählt aus der Gruppe bestehend aus Stickstoff und Sauerstoff enthalten kann,
ein aromatischer oder nichtaromatischer Bicyclus, der ein oder mehrere Heteroatome ausgewählt aus der Gruppe bestehend aus Schwefel und Sauerstoff enthalten kann;
R^{4.1} H, Methyl, Ethyl;
R^{4.2} Methyl, Ethyl, Phenyl;
bedeuten, sowie pharmakologisch verträgliche Salze, Diastereomere, Enantiomere, Racemate, Hydrate oder Solvate davon

6. Verbindungen der Formel **1,** nach einem der Ansprüche 1 bis 5, worin R⁴ Cl bedeutet, sowie pharmakologische verträgliche Salze, Diastereomere, Enantiomere, Racemate, Hydrate oder Solvate davon.

7. Verbindungen der Formel **1,** nach einem der Ansprüche 1 bis 6, worin
R³ eine Gruppe der allgemeinen Formel **1a,** worin
A ein fünf-, sechs- oder siebengliedriger heterocyclischer, aromatischer oder nichtaromatischer Ring, oder ein aromatischer oder nichtaromatischer, bicyclischer Ring bestehend aus acht, neun oder zehn Atomen, der gegebenenfalls ein, zwei oder drei Heteroatome enthält;
X NR^{3.2}, O, S;
Y C₁₋₄-Alkylen, das gegebenenfalls mit einem oder mehreren R^{3.3} substituiert sein kann
m 0, 1, 2 oder 3;
R^{3.1} jeweils unabhängig voneinander C₁₋₄-Alkyl, C₆₋₁₀.Aryl , COOR^{3.1.1}, CONR^{3.1.1}R^{3.1.2}, CN, NR^{3.1.1}R^{3.1.2}, NHCOR^{3.1.1}, OR^{3.1.1}, O-C₁₋₄-Haloalkyl, SO₂R^{3.1.1}, SO₂NH₂, Halogen;
R^{3.1.1} H, C₁-₆-Alkyl;
R^{3.1.2} H, C₁₋₆-Alkyl;
R^{3.2} H, C₁₋₆-Alkyl;
R^{3.3} jeweils unabhängig voneinander C₁-₆-Alkyl, C₁₋₆-Alkyl-OH, C₃₋₆-Cycloalkyl, O-C₁₋₆-Alkyl, COOH, COO-C₁₋₆-Alkyl, CONH₂;
R^{3.3} bildet gemeinsam mit einem oder zwei Kohlenstoffatomen von Y einen carbocyclischen Ring mit 3, 5 oder 6 Kohlenstoffatomen
bedeutet, sowie pharmakologisch verträgliche Salze, Diastereomere, Enantiomere, Racemate, Hydrate oder Solvate davon.

8. Verbindungen der Formel 1, nach einem der Ansprüche 1 bis 7, worin
R³ eine Gruppe der allgemeinen Formel **1a,** worin
A ein fünf-, sechs- oder siebengliedriger heterocyclischer, aromatischer oder nichtaromatischer Ring, der ein, zwei oder drei Heteroatome, unabhängig voneinander, ausgewählt aus der Gruppe bestehend aus Sauerstoff, Stickstoff und Schwefel, enthält; oder ein aromatischer oder nichtaromatischer, bicyclischer Ring bestehend aus acht, neun oder zehn Atomen, der gegebenenfalls ein, zwei oder drei Heteroatome, unabhängig voneinander, ausgewählt aus der Gruppe bestehend aus Sauerstoff, Stickstoff und Schwefel, enthält;
X NR^{3.2} O, S;
Y C₁₋₂-Alkylen, das gegebenenfalls mit einem oder mehreren R^{3.3} substituiert sein kann
m 0, 1, 2 oder 3;
R^{3.1} jeweils unabhängig voneinander C₁₋₄-Alkyl, C₆₋₁₀-Aryl, COOH, COO-C₁₋₄-Alkyl, CONH₂, CN, NH₂, NHCO-C₁₋₄-Alkyl, OH, O-C₁₋₄-Alkyl, O-C₁₋₄-Haloalkyl, SO₂-C₁₋₄-Akyl, SO₂NH₂, Halogen;
R^{3.2} H, C₁₋₄-Alkyl;
R^{3.3} H, C₁₋₄-Alkyl, C₁₋₄-Alkyl-OH, C₃₋₆-Cycloalkyl, O-C₁₋₄-Alkyl, COOH, COO-C1-4-Alkyl, CONH₂;
R^{3.3} bildet gemeinsam mit einem oder zwei Kohlenstoffatomen von Y einen carbocyclischen Ring mit 3, 5 oder 6 Kohlenstoffatomen
bedeutet, sowie pharmakologisch verträgliche Salze, Diastereomere, Enantiomere, Racemate, Hydrate oder Solvate davon.

9. Verbindungen der Formel **1**, nach Anspruch 8, worin
R^{3.1} jeweils unabhängig voneinander Methyl, Ethyl, Propyl, Ph, COOH, COOMe, CONH₂, CN, NH₂, NHCOMe, OH, OMe, OEt, OCF₃, OCHF₂, SO₂Me, SO₂NH₂, F, Cl, Br;
R³² H, C₁₋₄-Alkyl;
R³³ H, Methyl, Ethyl, Propyl, Butyl, CH₂OH CH₂CH₂OH, C(CH₂)₂OH, Cyclopropyl, COOH, COOMe, COOEt, COOPr, CONH₂, OMe, OEt, OPr;
R³³ bildet gemeinsam mit einem oder zwei Kohlenstoffatomen von Y einen carbocyclischen Ring mit 3, 5 oder 6 Kohlenstoffatomen
bedeutet, sowie pharmakologisch verträgliche Salze, Diastereomere, Enantiomere, Racemate, Hydrate oder Solvate davon.

10. Verbindungen der Formel **1**, nach Anspruch 9, worin
R^{3.1} Methyl, iso-Propyl, tert Butyl;
R^{3.2} H, Methyl;
R^{3.3} H, Methyl;
bedeutet, sowie pharmakologisch verträgliche Salze, Diastereomere, Enantiomere, Racemate, Hydrate oder Solvate davon.

11. Verbindungen der Formel **1** nach einem der Ansprüche 1 bis 10, worin
R³ eine Gruppen der allgemeinen Formel **1a**, worin
A ein gesättigter oder ungesättigter, mono- oder bicyclischer C₅₋₁₀-Heterocyclus mit 1, 2 oder 3 Heteroatomen ausgewählt aus der Gruppe bestehend aus N, O und S;
X NR^{3.2}, O;
Y C₁₋₂-Alkylen, gegebenenfalls substituiert mit einem oder mehreren R^{3.3}
m 0,1,2 oder 3;
R^{3.1} Methyl,
R^{3.2} H,
R^{3.3} H,
bedeuten, sowie pharmakologisch verträgliche Salze, Diastereomere, Enantiomere, Racemate, Hydrate oder Solvate davon.

12. Verbindungen der Formel **1** nach einem der Ansprüche 1 bis 11, worin
R³ eine Gruppe der allgemeinen Formel **1a,** worin
A Thiophen, Furan, Pyrazol, Pyridinl, Isoxazol, Thiazol, Benzimidazol, Benzo[b]thiophen, 2,3-Dihydrobenzo[1,4]dioxin, 1,2,3,4-Tetrahydronaphthalin, Oxazol, Tetrahydrofuran oder Tetrahydropyran;
X NR^{3.2}, O;
Y C₁₋₂-Alkylen, gegebenenfalls substituiert mit einem oder mehreren R^{3.3}
m 0,1,2 oder 3;
R^{3.1} Methyl, R^{3.2} H,
R^{3.3} H,
bedeuten, sowie pharmakologisch verträgliche Salze, Diastereomere, Enantiomere, Racemate, Hydrate oder Solvate davon.

13. Verbindungen der Formel **1** nach einem der Ansprüche 1 bis 12, worin
R¹ Azetidin
R² Piperazin
R³ eine Gruppe der allgemeinen Formel **1a**, worin
A Thiophen, Furan, Pyrazol, Pyridin, Isoxazol, Thiazol, Benzimidazol, Benzo[b]thiophen, 2,3-Dihydrobenzo[1,4]dioxin, 1,2,3,4-Tetrahydronaphthalin, Oxazol oder Phenyl, Tetrahydrofuran oder Tetrahydropyran,
X NR^{3.2}, O;
Y methylen oder ethylen
m 0 ,1, 2 oder 3;
R^{3.1} jeweils unabhängig voneinander Methyl, Ethyl, Phenyl, Halogen, COOR^{3.1.1}, CONR^{3.1.1}R^{3.1.2}, CN, NR^{3.1.1}R^{3.1.2}, NHCOR^{3.1.1}, OR^{3.1.1}, O-C₁₋3-Haloalkyl, SO₂R^{3.1.1}, SO₂NH₂, C₁₋₃-Haloalkyl, wobei R^{3.1.1} und R^{3.1.2} unabhängig voneinander H, Methyl, Ethyl, Propyl sein können
R^{3.2} H oder Methyl
bedeuten, sowie pharmakologisch verträgliche Salze, Diastereomere, Enantiomere, Racemate, Hydrate oder Solvate davon.

14. Verbindungen der Formel **1** nach einem der Ansprüche 1 bis 13, worin
R¹ Azetidin,
R² Piperazin,
R³ O-C₁₋₂₋alkylen-phenyl oder NH-C₁₋₂-alkylen-phenyl und
R⁴ Cl bedeutet, sowie pharmakologisch verträgliche Salze, Diastereomere,
Enantiomere, Racemate, Hydrate oder Solvate davon.

15. Verbindungen der Formel **2** 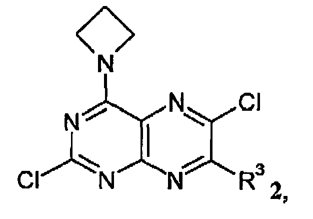 worin
R³ eine Gruppe der Formel **1a,** 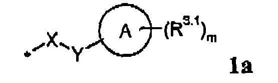 worin
A ein Ring ausgewählt aus der Gruppe bestehend aus einem monocyclischen, heterocyclischen Ring, einem bicyclischen Ring, der gegebenenfalls ein oder mehrere Heteroatome enthält, einem C₆₋₁₀-Aryl und einem C₅₋₁₀-Heteroaryl
X NR^{3.2}, O, S;
Y C₁₋₄-Alkylen, das gegebenenfalls mit einem oder mehreren R^{3.3} substituiert sein kann,
m 0, 1, 2 oder 3
R^{3.1} worin jeweils unabhängig voneinander ausgewählt sind aus der Gruppe bestehend aus C₁₋₆-Alkyl, C₆₋₁₀-Aryl, COOR^{3.1.1}, CONR^{3.1.1}R^{3.1.2}, CN, NR^{3.1.1}R^{3.1.2}, NHCOR^{3.1.1}, OR^{3.1.1}, O-C₁₋₆-Haloalkyl, SO₂R^{3.1.1}, SO₂NR₂, Halogen, C₁₋₆-Haloalkyl, C₁₋₆-alkyl-CONR^{3.1.1}R^{3.1.2}, C₁₋₆-alkyl NR^{3.1.1}R^{3.1.2}, C₁₋₆-alkyl-CONH₂, O-C₁₋₆₋akylen-NH₂, O-C₃₋₆-Cycloalkyl, O-C₁₋₄-Alkylen-C₃₋₆-cycloalkyl,O-C₁₋₄-Alkylen-CONH₂ und SO₂NR^{3.1.1}R^{3.1.2},
oder
R^{3.1} gemeinsam mit zwei Atomen von A einen 5 oder 6-gliedrigen carbocyclischen Ring oder einen 5 oder 6-gliedrigen heterocyclischen Ring bildet, der gegebenenfalls ein oder mehrere Heteroatome ausgewählt aus der Gruppe bestehend aus Sauerstoff und Stickstoff enthalten kann,
worin
R^{3.1.1} H, C₁₋₆-Alkyl;
R^{3.1.2} H, C₁₋₆-Alkyl;
und
R^{3.2} H, C₁₋₆-Alkyl;
und worin
R^{3.3} oder jeweils unabhängig voneinander H, C₁₋₆-Alkyl, C₁₋₆-Alkyl-OH, C₃₋₆-Cycloalkyl, C₃₋₆Cycloalkyl-OH, O-C₁₋₆-Alkyl, COOR^{3.1.1}, COO-C₁₋₆-Alkyl oder CONR^{3.1.1}R^{3.1.2} bedeuten, worin
R^{3.3} gemeinsam mit einem oder zwei Kohlenstoffatomen von Y einen carbocyclischen Ring mit 3, 4, 5 oder 6 Kohlenstoffatomen bildet,
sowie deren pharmakologisch verträgliche Salze, Diastereomere, Enantiomere, Racemate, Hydrate oder Solvate davon.

16. Arzneimittel enthaltend eine der Verbindungen nach einem der Ansprüche 1 bis 14.

17. Verwendung von Verbindungen nach einem der Ansprüche 1 bis 14 zur Herstellung eines Medikaments zur Behandlung von Atemwegs- oder gastrointestinalen Beschwerden oder Erkrankungen, wie auch entzündliche Erkrankungen der Gelenke, der Haut oder der Augen, Krebserkrankungen, sowie von Erkrankungen des periphären oder zentralen Nervensystems.

18. Verwendung von Verbindungen nach einem der Ansprüche 1 bis 14 zur Herstellung eines Medikament zur Behandlung von entzündlichen und obstruktiven Erkrankungen wie COPD, chronischer Sinusitis, Asthma, Morbus Crohn, Colitis ulcerosa.

19. Verwendung, nach einem der Ansprüche 17 bis 18, wobei Nebenwirkungen der Behandlung reduziert sind,

20. Verwendung, nach einem der Ansprüche 17 bis 18, wobei die reduzierten Nebenwirkungen ausgewählt aus der Gruppe Emesis und Nausea sind.

## Claims

1. Compounds of formula 1, 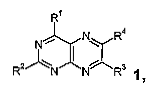 wherein
R¹ denotes a saturated or unsaturated, four-membered heterocyclic group, which contains a nitrogen atom and may optionally contain a further atom selected from among nitrogen, sulphur and oxygen;
R² denotes halogen, OR^{2.1} SR^{2.1}, NR^{2.1}R^{2.2},
where
R^{2.1} denotes H, C₁₋₄-alkyl, C₆₋₁₀-aryl, C₇₋₁₁- aralkyl;
R^{2.2} denotes H, C₁₋₄-alkyl, C₆₋₁₀-aryl, C₇₋₁₁- aralkyl;
or
R² denotes a group selected from among C₆₋₁₀-aryl, C₆₋₁₀-heteroaryl and a five-, six- or seven-membered heterocyclic group, which contains a nitrogen atom and which may optionally contain a further atom selected from among nitrogen, sulphur and oxygen, while this group may optionally be substituted by a group selected from among C₁₋₆-alkyl, O-C₁₋₆-alkyl, C₃₋₆-cycloalkyl, C₇₋₁₁- aralkyl and N(C₁₋₄-alkyl),
R³ denotes a group of formula **1a**, 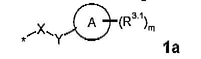 wherein
A denotes a ring selected from among a monocyclic, heterocyclic ring, a bicyclic ring which optionally contains one or more heteroatoms, a C₆₋₁₀-aryl and a C₅₋₁₀-heteroaryl,
X denotes NR^{3.2}, O, S;
Y denotes C₁₋₄-alkylene, which may optionally be substituted by one or more R^{3.3},
m denotes 0, 1, 2 or 3
R^{3.1} each independently of one another denote C₁₋₆-alkyl, C₆₋₁₀-aryl, COOR^{3.1.1}, CONR^{3.1.1}R^{3.1.2}, CN, NR^{3.1.1}R^{3.1.2}, NHCOR^{3.1.1}, OR^{3.1.1}, O-C₁₋₆-haloalkyl, SO₂R^{3.1.1}, SO₂NH₂, halogen, C₁₋₆-haloalkyl, C₁₋₆-alkyl-CONR^{3.1.1}R^{3.1.2}, C₁₋₆-alkyl-NR^{3.1.1},R^{3.7.2}, C₁₋₆-alkyl-CONH₂, O-C₁₋₆-alkylene-NH₂, O-C₃₋₆-cycloalkyl, O-C₁₋₄-alkylene-C₃₋₆-cycloalkyl,O-C₁₋₄-alkylene-CONH₂, SO₂NR^{3.1.1}R^{3.1.1};
or
R^{3.1} together with two atoms of A forms a 5- or 6-membered carbocyclic ring or a 5- or 6-membered heterocyclic ring which may optionally contain one or more heteroatoms selected from among oxygen and nitrogen,
wherein
R^{3.1.1} denotes H, C₁₋₆-alkyl;
R^{3.1.2} denotes H, C₁₋₆-alkyl;
and
R^{3.2} denotes H, C₁₋₆-alkyl;
and wherein
R^{3.3} each independently of one another denote H, C₁₋₆-alkyl, C₁₋₆-alkyl-OH, C₃₋₆-cycloalkyl, C₃₋₆-cycloalkyl-OH, O-C₁₋₆-alkyl, COOR^{3.1.1}, COO-C₁₋₆-alkyl, CONR^{3.1.1}R^{3.1.2};
or R^{3.3} together with one or two carbon atoms of Y forms a carbocyclic ring with 3, 4, 5 or 6 carbon atoms,
R⁴ denotes a group selected from among halogen, C₁₋₆-alkyl, C₂₋₆-alkenyl, C₂₋₆-alkynyl, C₃₋₆-cycloalkyl, C₃₋₆-cycloalkenyl, OR^{4.1}, SR^{4.1} C₁₋₆-haloalkyl, NR^{4.1}R^{4.2} or a group selected from among C₆₋₁₀-aryl, 3-10-membered heterocyclic group and C₅₋₁₀-heteroaryl, which may optionally be substituted by one or more groups selected from among C₁₋₆-alkyl, C₁₋₆-haloalkyl, CN, O-C₁₋₆-alkyl, halogen,
R^{4.1} denotes H, C₁₋₆-alkyl, C₆₋₁₀-aryl, C₇₋₁₁-aralkyl;
R^{4.12} denotes H, C₁₋₆-alkyl, C₆₋₁₀-aryl, C₇₋₇₇- aralkyl;
and pharmacologically acceptable salts, diastereomers, enantiomers, racemates, hydrates or solvates thereof.

2. Compounds of formula **1**, according to claim 1, wherein
R¹ denotes an azetidine ring
and pharmacologically acceptable salts, diastereomers, enantiomers, racemates, hydrates or solvates thereof.

3. Compounds of formula **1**, according to claim 1 or 2, wherein
R¹ denotes an azetidine ring,
R² denotes a five- or six-membered heterocyclic group, which contains one or two nitrogen atoms;
and pharmacologically acceptable salts, diastereomers, enantiomers, racemates, hydrates or solvates thereof.

4. Compounds of formula **1**, according to one of claims 1 to 3, wherein
R¹ is an azetidine ring;
R² is a six-membered heterocyclic group which contains two nitrogen atoms;
and pharmacologically acceptable salts, diastereomers, enantiomers, racemates, hydrates or solvates thereof.

5. Compounds of formula **1**, according to one of claims 1 to 4, wherein
R⁴ denotes a group selected from among Cl, F, Br, methyl, ethyl, propyl, 2-methylpropyl, cyclopropyl, cyclohexyl, methoxy, CF₃, NR^{4.1}R^{4.2},
C₆₋₁₀-aryl, optionally substituted by one or more groups selected from among methyl, CF₃, CN, methoxy, fluorine, chlorine,
a five- or six-membered heterocyclic ring, which may contain one or more heteroatoms selected from among nitrogen and oxygen,
a five-membered heterocyclic aromatic group, which may contain one or more groups selected from among nitrogen and oxygen,
an aromatic or non-aromatic bicyclic group, which may contain one or more heteroatoms selected from among sulphur and oxygen contain may;
R^{4.1} denotes H, methyl, ethyl;
R^{4.2} denotes methyl, ethyl, phenyl;
and pharmacologically acceptable salts, diastereomers, enantiomers, racemates, hydrates or solvates thereof.

6. Compounds of formula **1**, according to one of claims 1 to 5, wherein
R⁴ denotes Cl
and pharmacologically acceptable salts, diastereomers, enantiomers, racemates, hydrates or solvates thereof.

7. Compounds of formula **1**, according to one of claims 1 to 6, wherein
R³ denotes a group of general formula **1a**, wherein
A denotes a five-, six- or seven-membered heterocyclic, aromatic or non-aromatic ring, or an aromatic or non-aromatic, bicyclic ring consisting of eight, nine or ten atoms, which optionally contains one, two or three heteroatoms;
X denotes NR^{3.2}, O, S;
Y denotes C₁₋₄-alkylene, which may optionally be substituted by one or more R^{3.3}
m denotes 0, 1, 2 or 3;
R^{3.1} each independently of one another denote C₁₋₄-alkyl, C₆₋₁₀-aryl, COOR^{3.1.1}, CONR^{3.1.1}R^{3.1.2}, CN, NR^{3.1.1}R^{3.1.2}_{,} NHCOR^{3.1.1}, OR^{3.1.1}, O-C₁₋₄-haloalkyl, SO₂R^{3.1.1}, SO₂NH₂, halogen;
R^{3.1.1} denotes H, C₁₋₆-alkyl;
R^{3.1. 2} denotes H, C₁₋₆-alkyl;
R^{3.2} denotes H, C₁₋₆-alkyl;
R^{3.3} each independently of one another denote C₁₋₆-alkyl, C₁₋₆-alkyl-OH, C₃₋₆-cycloalkyl, O-C₁₋₆-alkyl, COOH, COO-C₁₋₆-alkyl, CONH₂;
R^{3.3} together with one or two carbon atoms of Y forms a carbocyclic ring with 3, 5 or 6 carbon atoms
and pharmacologically acceptable salts, diastereomers, enantiomers, racemates, hydrates or solvates thereof.

8. Compounds of formula **1**, according to one of claims 1 to 7, wherein
R³ denotes a group of general formula **1a**, wherein
A denotes a five-, six- or seven-membered heterocyclic, aromatic or non-aromatic ring, which contains one, two or three heteroatoms, independently of one another, selected from among oxygen, nitrogen and sulphur; or an aromatic or non-aromatic bicyclic ring consisting of eight, nine or ten atoms, which optionally contains one, two or three heteroatoms, independently of one another, selected from among oxygen, nitrogen and sulphur;
X denotes N^{3.2}, O, S;
Y denotes C₁₋₂-alkylene, which may optionally be substituted by one or more R^{3.3}
m denotes 0, 1, 2 or 3;
R^{3.1} each independently of one another denote C₁₋₄-alkyl, C₆₋₁₀-aryl, COOH, COO-C₁₋₄-alkyl, CONH₂, CN, NH₂, NHCO-C₁₋₄-alkyl, OH, O-C₁₋₄-alkyl, O-C₁₋₄-haloalkyl, SO₂-C₁₋₄-alkyl, SO₂NH₂, halogen;
R^{3.2} denotes H, C₁₋₄-alkyl;
R^{3.3} denotes H, C₁₋₄-alkyl, C₁₋₄-alkyl-OH, C₃₋₆-cycloalkyl, O-C₁₋₄-alkyl, COOH, COO-C₁₋₄-alkyl, CONH₂,
R^{3.3} together with one or two carbon atoms of Y forms a carbocyclic ring with 3, 5 or 6 carbon atoms,
as well as pharmacologically acceptable salts, diastereomers, enantiomers, racemates, hydrates or solvates thereof.

9. Compounds of formula **1**, according to claim 8, wherein
R^{3.1} each independently of one another denote methyl, ethyl, propyl, Ph, COOH, COOMe, CONH₂, CN, NH₂, NHCOMe, OH, OMe, OEt, OCF₃, OCHF₂, SO₂Me, SO₂NH₂, F, Cl, Br;
R^{3.2} denotes H, C₁₋₄-alkyl;
R^{3.3} denotes H, methyl, ethyl, propyl, butyl, CH₂OH, CH₂CH₂OH, C(CH₂),OH, cyclopropyl, COOH, COOMe, COOEt, COOPr, CONH₂, OMe, OEt, OPr;
R^{3.3} together with one or two carbon atoms of Y forms a carbocyclic ring with 3, 5 or 6 carbon atoms
and pharmacologically acceptable salts, diastereomers, enantiomers, racemates, hydrates or solvates thereof.

10. Compounds of formula **1**, according to claim 9, wherein
R^{3.1} denotes methyl, iso-propyl, tert-butyl;
R^{3.2} denotes H, methyl;
R^{3.3} denotes H, methyl;
and pharmacologically acceptable salts, diastereomers, enantiomers, racemates, hydrates or solvates thereof.

11. Compounds of formula **1** according to one of claims 1 to 10, wherein
R³ denotes a group of general formula **1a**, wherein
A denotes a saturated or unsaturated, mono- or bicyclic C₅₋₁₀ heterocycle with 1, 2 or 3 heteroatoms selected from among N, O and S;
X denotes NR^{3.2}, O;
Y denotes C₁₋₂-alkylene, optionally substituted by one or more R^{3.3}
m denotes 0, 1, 2 or 3;
R^{3.1} denotes methyl,
R^{3.2} denotes H,
R^{3.3} denotes H,
and pharmacologically acceptable salts, diastereomers, enantiomers, racemates, hydrates or solvates thereof.

12. Compounds of formula **1** according to one of claims 1 to 11, wherein
R³ denotes a group of general formula **1a**, wherein
A denotes thiophene, furan, pyrazole, pyridine, isoxazole, thiazole, benzimidazole, benzo[b]thiophene, 2,3-dihydrobenzo[1,4]dioxin, 1,2,3,4-tetrahydronaphthalene, oxazole, tetrahydrofuran or tetrahydropyran;
X denotes NR^{3.2}, O;
Y denotes C₁₋₂-alkylene, optionally substituted by one or more R^{3.3}
m denotes 0, 1, 2 or 3;
R^{3.1} denotes methyl,
R^{3.2} denotes H,
R^{3.3} denotes H,
and pharmacologically acceptable salts, diastereomers, enantiomers, racemates, hydrates or solvates thereof.

13. Compounds of formula 1 according to one of claims 1 to 12, wherein
R¹ denotes azetidine
R² denotes piperazine
R³ denotes a group of general formula **1a**, wherein
A denotes thiophene, furan, pyrazole, pyridine, isoxazole, thiazole, benzimidazole, benzo[b]thiophene, 2,3-dihydrobenzo[1,4]dioxin, 1,2,3,4-tetrahydronaphthalene, oxazole or phenyl, tetrahydrofuran or tetrahydropyran;
X denotes NR^{3.2}, O;
Y denotes methylene or ethylene
m denotes 0, 1, 2 or 3;
R^{3.1} each independently of one another denote methyl, ethyl, phenyl, halogen, COOR^{3.1.1}, CONR^{31.1}R^{3.1.2}, CN, NR^{3.1.1}R^{3.1.2}, NHCOR^{3.1.1}, OR^{3.1.1}, O-C₁₋₃-haloalkyl, SO₂R^{3.1.1}, SO₂NH₂, C₁₋₃-haloalkyl, wherein R^{3.1.1} and R^{3.1.2} independently of one another may be H, methyl, ethyl, propyl;
R^{3.2} denotes H or methyl
and pharmacologically acceptable salts, diastereomers, enantiomers, racemates, hydrates or solvates thereof.

14. Compounds of formula 1 according to one of claims 1 to 13, wherein
R¹ denotes azetidine,
R² denotes piperazine,
R³ denotes O-C₁₋₂-alkylene-phenyl or NH-C₁₋₂-alkylene-phenyl and
R⁴ denotes Cl
and pharmacologically acceptable salts, diastereomers, enantiomers, racemates, hydrates or solvates thereof.

15. Compounds of formula 2 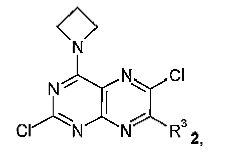 wherein
R³ denotes a group of formula 1a, 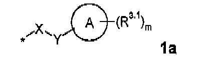 wherein
A denotes a ring selected from among a monocyclic, heterocyclic ring, a bicyclic ring which optionally contains one or more heteroatoms, a C₆₋₁₀-aryl and a C₆₋₁₀-heteroaryl
X denotes NR^{3.2}, O, S;
Y denotes C₁₋₄-alkylene, which may optionally be substituted by one or more R^{3.3},
m denotes 0, 1, 2 or 3
R^{3.1} are each independently of one another selected from among C₁₋₆-alkyl, C₆₋₁₀-aryl, COOR^{3.1.1}, CONR^{3.1.1}R^{3.1.2}, CN, NR^{3.1.1}R^{3.1.2}, NHCOR^{3.1.1}, OR^{3.1.1}, O-C₁₋₆-haloalkyl, SO₂R^{3.1.1}, SO₂NH₂, halogen, C₁₋₆-haloalkyl, C₁₋₆-alkyl-CONR^{3.1.1}R^{3.1.2}, C₁₋₆-alkyl-NR^{3.1.1}R^{3.1.2}, C₁₋₆-alkyl-CONH₂, O-C₁₋₆-alkylone-NH₂, O-C₃₋₆-cycloalkyl, -C₁₋₄-alkylene-C₃₋₆-cycloalkyl, O-C₁₋₄-alkylene-CONH₂ and SO₂NR^{3.1.1}R^{3.1.2};
or
wherein
R^{3.1} together with two atoms of A forms a 5- or 6-membered carbocyclic ring or a 5- or 6-membered heterocyclic ring, which may optionally contain one or more heteroatoms selected from among oxygen and nitrogen,
wherein
R^{3.1.1} denotes H, C₁₋₆-alkyl;
R^{3.1.2} denotes H, C₁₋₆-alkyl;
and
R^{3.2} denotes H, C₁₋₆-alkyl;
and wherein
R^{3.3} each independently of one another denote H, C₁₋₆-alkyl, C₁₋₆-alkyl-OH, C₃₋₆-cycloalkyl, C₃₋₆-cycloalkyl-OH, O-C₁₋₆-alkyl, COOR^{3.1.1}, COO-C₁₋₆-alkyl or CONR^{3.1.1}R^{3.1.2} or
wherein
R^{3.3} together with one or two carbon atoms of Y forms a carbocyclic ring with 3, 4, 5 or 6 carbon atoms,
as well as the pharmacologically acceptable salts, diastereomers, enantiomers, racemates, hydrates or solvates.

16. Medicaments containing one of the compounds according to one of claims 1 to 14.

17. Use of compounds according to one of claims 1 to 14 for preparing a medicament for the treatment of respiratory or gastrointestinal complaints or diseases, such as inflammatory diseases of the joints, skin or eyes, cancers, and diseases of the peripheral or central nervous system.

18. Use of compounds according to one of claims 1 to 14 for preparing a medicament for the treatment of inflammatory and obstructive diseases such as COPD, chronic sinusitis, asthma, Crohn's disease and ulcerative colitis.

19. Use according to one of claims 17 to 18, wherein side effects of the treatment are reduced.

20. Use according to one of claims 17 to 18, wherein the reduced side effects are selected from among emesis and nausea.

## Revendications

1. Composé de formule 1 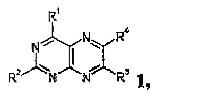 dans laquelle
R¹ peut contenir un hétérocycle saturé ou insaturé à quatre chaînons qui contient un atome d'azote et éventuellement un autre atome choisi dans le groupe comprenant un atome d'azote, de soufre et d'oxygène ;
R² est un atome d'halogène, OR^{2,1}, SR^{2,1}, NR^{2,1}R^{2,2}, dans laquelle
R^{2,1} est H, un groupe alkyle en C₁ à C₄, aryle en C₆ à C₁₀, aralkyle en C₇ à C₁₁ ;
R^{2,2} est H, un groupe alkyle en C₁ à C₄, aryle en C₆ à C₁₀, aralkyle en C₇ à C₁₁ ;
ou
R² est un radical choisi dans le groupe comprenant un groupe aryle en C₆ à C₁₀, hétéroaryle en C₅ à C₁₀ et un hétérocycle de cinq, six ou sept chaînons qui contient un atome d'azote et qui peut contenir éventuellement un autre atome choisi dans le groupe comprenant un atome d'azote, de soufre et d'oxygène, ce radical pouvant être substitué éventuellement par un radical choisi dans le groupe comprenant un groupe alkyle en C₁ à C₆, O-alkyle en C₁ à C₆, cycloalkyle en C₃ à C₆, aralkyl; en C₇ à C₁₁ et N (alkyle en C₁ à C₄) ₂,
R² est un groupe de formule 1a, 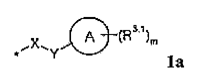 dans laquelle
A est un cycle choisi dans le groupe comprenant un cycle monocyclique, hétérocyclique, un cycle bicyclique qui contient éventuellement un ou plusieurs hétéroatomes, un groupe aryle en C₆ à C₁₀ et un groupe hétéroaryle en C₅ à C₁₀
X est NR^{3,2}, O, S ;
Y est un groupe alkylène en C₁ à C₄ qui peut être substitué éventuellement par un ou plusieurs R^{3.3}
m désigne 0, 1, 2 ou 3
les R^{3,1}, indépendamment les uns des autres sont un groupe alkyle en C₁ à C₆, aryle en C₆ à C₁₀, COOR^{3,1,1}, CONR^{3,1,1}R^{3,1,2}, CN, NR^{3,1,1}R^{3,1,2}, NHCOR^{3,1,1}, OR^{3,1,1}, O-halogénoalkyle en C₁ à C₆, SO₂R3,1,1, *SO₂NH₂, un atome d'halogène, un groupe halogénoalkyle en C₁ à C₆, alkyle en C₁ à C₆-CONR^{3,1,1}R^{3,1,2}, alkyle en C₁ à C₆-NR^{3,1,1}R^{3,1,2}, alkyle en C₁ à C₆-CONH₂, O-alkylène en C₁ à C₆-NH₂, O-cycloalkyle en C₃ à C₆, O-alkylène en C₁ à C₄-cycloalkyle en C₃ à C₆, O-alkylène en C₁ à C₄-CONH₂, SO₂NR^{3,1,1}R^{3,1,2};
ou
R^{3,1} forme conjointement avec deux atomes de A, un cycle carbocyclique de 5 ou 6 chaînons ou un cycle hétérocyclique de 5 à 6 chaînons qui peut contenir éventuellement un ou plusieurs hétéroatomes choisis dans le groupe comprenant un atome d'oxygène et d'azote,
dans laquelle
R^{3,1,1} est H, un groupe alkyle en C₁ à C₆ ;
R^{3,1,2} est H, un groupe alkyle en C₁ à C₆ ;
et
R^{3,2} est H, un groupe alkyle en C₁ à C₆ ;
et dans laquelle
les R^{3,3} sont respectivement, indépendamment les uns des autres, H, un groupe alkyle en C₁ à C₆, alkyle en C₁ à C₆-OH, cycloalkyle en C₃ à C₆, cycloalkyle en C₃ à C₆-OH, O-alkyle en C₁ à C₆, COOR^{3,1,1}, COO-alkyle en C₁ à C₆, CONR^{3,1,1}R^{3,1,2} ;
ou
R^{3,3} forme conjointement avec un ou deux atomes de carbone de Y, un cycle carbocyclique comportant 3, 4, 5 ou 6 atomes de carbone,
R⁴ est un radical choisi dans le groupe comprenant un atome d'halogène, un groupe alkyle en C₁ à C₆, alcényle en C₂ à C₆, alcinyle en C₂ à C₆, cycloalkyle en C₃ à C₆, cycloalcényle en C₃ à C₆, OR^{4,1}, SR^{4,1}, halogénoalkyle en C₁ à C₆, NR^{4,1}R^{4,2} ou un radical choisi dans le groupe comprenant un groupe aryle en C₆ à C₁₀, un hétérocycle de 3 à 10 chaînons et un groupe hétéroaryle en C₅ à C₁₀, qui peut être substitué éventuellement par un ou plusieurs radicaux choisis dans le groupe comprenant un groupe alkyle en C₁ à C₆, halogénoalkyle en C₁ à C₆, CN, O-alkyle en C₁ à C₆, un atome d'halogène,
R^{4,1} est H, un groupe alkyle en C₁ à C₆, aryle en C₆ à C₁₀, aralkyle en C₇ à C₁₁ ;
R^{4,2} est H, un groupe alkyle en C₁ à C₆, aryle en C₆ à C₁₀, aralkyle en C₇ à C₁₁ ;
et leurs sels, diastéréomères, énantiomères, racémates, hydrates ou solvates pharmacologiquement acceptables.

2. Composés de formule 1, selon la revendication 1, dans laquelle
R¹ est un cycle azétidine
et leurs sels, diastéréoméres, énantiomères, racémates, hydrates ou solvates pharmacologiquement acceptables.

3. Composés de formule 1, selon la revendication 1 ou 2, dans laquelle
R¹ est un cycle azétidine,
R² est un hétérocycle de cinq ou six chaînons qui contient un ou deux atomes d'azote,
et leurs sels, diastéréomères, énantiomères, racémates, hydrates ou solvates pharmacologiquement acceptables.

4. Composés de formule 1, selon l'une des revendications 1 à 3, dans laquelle
R¹ est un cycle azétidine,
R² est un hétérocycle de six chaînons qui contient deux atomes d'azote,
et leurs sels, diastéréoinères, énantiomères, racémates, hydrates ou solvates pharmacologiquement acceptables.

5. Composés de formule 1, selon l'une des revendications 1 à 4, dans laquelle
R⁴ est un radical choisi dans le groupe comprenant Cl, F, Br, un groupe méthyle, éthyle, propyle, 2-méthylpropyle, cyclopropyle, cyclohexyle, méthoxy, CF₃, NR^{4,1}R^{4,2}, un groupe aryle en C₆ à C₁₀, éventuellement substitué par un ou plusieurs radicaux choisis dans le groupe comprenant un groupe méthyle, CF₃, CN, méthoxy, un atome de fluor, de chlore,
un cycle hétérocyclique de cinq ou six chaînons qui peut contenir un ou plusieurs hétéroatomes choisis dans le groupe comprenant un atome d'azote et d'oxygène,
un composé aromatique hétérocyclique à cinq chaînons qui peut contenir un ou plusieurs radicaux choisis dans le groupe comprenant un atome d'azote et d'oxygène,
un bicycle aromatique ou non aromatique qui peut contenir un ou plusieurs hétéroatomes choisis dans le groupe comprenant un atome de soufre et d'oxygène ;
R^{4,1} est H, un groupe méthyle, éthyle ;
R^{4,2} est un groupe méthyle, éthyle, phényle ;
et leurs sels, diastéréomères, énantiomères, racémates, hydrates ou solvates pharmacologiquement acceptables.

6. Composés de formule 1, selon l'une des revendications 1 à 5, dans laquelle
R⁴ désigne Cl, et ses sels, diastéréomères, énantiomères, racémates, hydrates ou solvates pharmacologiquement acceptables.

7. Composés de formule 1, selon l'une des revendications 1 à 6, dans laquelle
R³ est un groupe de formule générale 1a, dans laquelle
A est un cycle hétérocyclique, aromatique ou non aromatique de cinq, six ou sept chaînons ou
un cycle bicyclique aromatique ou non aromatique constitué de huit, neuf ou dix atomes qui contient éventuellement un, deux ou trois hétéroatomes ;
X est NR^{3,2}, 0, S ;
Y est un groupe alkylène en C₁ à C₄ qui peut être substitué éventuellement par un ou plusieurs R^{3,3}
m est 0, 1, 2 ou 3 ;
les R^{3,1} sont respectivement, indépendamment les uns des autres, un groupe alkyle en C₁ à C₄, aryle en C₆ à C₁₀, COOR^{3,1,1}, CONR^{3,1,1}R^{3,1,2}, CN, NR^{3,1,1}R^{3,1,2}, NHCOR^{3,1,1} OR^{3,1,1}, O-halogénoalkyle en C₁ à C₄, SO₂R^{3,1,1}, SO₂NH₂, un atome d'halogène ;
R^{3,1,1} est H, un groupe alkyle en C₁ à C₆ ;
R^{3,1,2} est H, un groupe alkyle en C₁ à C₆ ;
R^{3,2} est H, un groupe alkyle en C₁ à C₆,
les R^{3,3} sont respectivement, indépendamment les uns des autres, un groupe alkyle en C₁ à C₆, alkyle en C₁ à C₆-OH, cycloalkyle en C₃ à C₆, O-alkyle en C₁ à C₆, COOH, COO-alkyle en C₁ à C₆, CONH₂ ;
R^{3,3} forme conjointement avec un ou deux atomes de carbone de Y, un cycle carbocyclique comportant 3, 5 ou 6 atomes de carbone ;
et leurs sels, diastéréomères, énantiomères, racémates, hydrates ou solvates pharmacologiquement acceptables.

8. Composés de formule 1, selon l'une des revendications 1 à 7, dans laquelle
R³ est un groupe de formule générale 1a, dans laquelle
A est un cycle hétérocyclique, aromatique ou non aromatique de cinq, six ou sept chaînons qui contient un, deux ou trois hétéroatomes indépendamment les uns des autres, choisis dans le groupe comprenant un atome d'oxygène, d'azote et de soufre ; ou
un cycle bicyclique aromatique ou non aromatique constitué de huit, neuf ou dix atomes qui contient éventuellement un, deux ou trois hétéroatomes, indépendamment les uns des autres, choisis dans le groupe comprenant un atome d'oxygène, d'azote et de soufre ;
X est NR^{3,2}, O, S ;
Y est un groupe alkylène en C₁ à C₂ qui peut être substitué éventuellement par un ou plusieurs R^{3,3}
m est 0, 1, 2 ou 3 ;
les R^{3,1} sont respectivement, indépendamment les uns des autres, un groupe alkyle en C₁ à C₄, aryle en C₆ à C₁₀, COOH, COO-alkyle en C₁ à C₄, CONH₂, CN, NH₂, NHCO-alkyle en C₁ à C₄, OH, O-alkyle en C₁ à C₄, O-halogénoalkyle en C₁ à C₄, SO₂-alkyle en C₁ à C₄, SO₂NH₂, un atome d'halogène ;
R^{3,2} est H, un groupe alkyle en C₁ à C₄ ;
les R^{3,3} sont H, un groupe alkyle en C₁ à C₄, allyle en C₁ à C₄-OH, cycloalkyle en C₃ à C₆, O-alkyle en C₁ à C₄, COOH, COO-alkyle en C₁ à C₄, CONH₂ ;
R^{3,3} forme conjointement avec un ou deux atomes de carbone de Y, un cycle carbocyclique comportant 3, 5 ou 6 atomes de carbone ;
et leurs sels, diastéréomères, énantiomères, racémates, hydrates ou solvates pharmacologiquement acceptables.

9. Composés de formule 1, selon la revendication 8, dans laquelle
les R^{3'1} sont respectivement, indépendamment les uns des autres, un groupe méthyle, éthyle, propyle, Ph, COOH, COOMe, CONH₂, CN, NH₂, NHCOMe, OH, OMe, OEt, OCF₃, OCHF₂, SO₂Me, SO₂NH₂, F, Cl, Br ;
R^{3,2} est H, un groupe alkyle en C₁ à C₄ ;
R^{3,3} est H, un groupe méthyle, éthyle, propyle, butyle, CH₂OH, CH₂CH₂OH, C(CH₂)₂OH, cyclopropyle, COOH, COOMe, COOEt, COOPr, CONH₂, OMe, OEt, OPr ;
R^{3,3} forme conjointement avec un ou deux atomes de carbone de Y, un cycle carbocyclique comportant 3, 5 ou 6 atomes de carbone ;
et leurs sels, diastéréomères, énantiomères, racémates, hydrates ou solvates pharmacologiquement acceptables.

10. Composés de formule 1, selon la revendication 9, dans laquelle
R^{3,1} est un groupe méthyle, iso-propyle, tert-butyle ;
R^{3,2} est H, un groupe méthyle ;
R^{3,3} est H, un groupe méthyle ;
et leurs sels, diastéréomères, énantioméres, racémates, hydrates ou solvates pharmacologiquement acceptables.

11. Composés de formule 1, selon l'une des revendications 1 à 10, dans laquelle
R³ est un groupe de formule générale 1a, dans laquelle
A est un hétérocycle mono- ou bicyclique en C₅ à C₁₀, saturé ou insaturé, comportant 1, 2 ou 3 hétéroatomes choisis dans le groupe comprenant N, 0 et S ;
X est NR^{3,2}, O ;
Y est un groupe alkylène en C₁ à C₂, éventuellement substitué par un ou plusieurs R^{3,3}
m est 0, 1, 2 ou 3 ;
R^{3'1} est un groupe méthyle, R^{3,2} est H,
R^{3,3} est H,
et leurs sels, diastéréomères, énantiomères, racémates, hydrates ou solvates pharmacologiquement acceptables.

12. Composés de formule 1 selon l'une des revendications 1 à 11, dans laquelle
R³ est un groupe de formule générale 1a, dans laquelle
A est un groupe thiophène, furane, pyrazole,
pyridine, isoxazole, thiazole, benzimidazole, benzo[b]thiophéne, 2,3-dihydrobenzo[1,4]dioxine, 1,2,3,4-tétrahydronaphtaline, oxazole, tétrahydrofurane ou tétrahydropyrane ;
X est NR^{3,2}, O ;
Y est un groupe alkylène en C₁ à C₂, éventuellement substitué par un ou plusieurs R^{3,3},
m est 0, 1, 2 ou 3 :
R^{3,1} est un groupe méthyle,
R^{3,2} est H,
R^{3,3} est H,
et leurs sels, diastéréomères, énantiomères, racémates, hydrates ou solvates pharmacologiquement acceptables.

13. Composés de formule 1 selon l'une des revendications 1 à 12, dans laquelle
R¹ est un groupe azétidine,
R² est un groupe pipérazine,
R³ est un groupe de formule générale 1a, dans laquelle
A est un groupe thiophène, furane, pyrazole, pyridine, isoxazole, thiazole, benzimidazole, benzo[b]thiophène, 2,3-dihydrobenzo[1,4]dioxine, 1,2,3,4-tétrahydronaphtaline, oxazole ou phényle, tétrahydrofurane ou tétrahydropyrane ;
X est NR^{3,2}, O ;
Y est un groupe méthylène ou éthylène,
m est 0, 1, 2 ou 3 ;
les R^{3,1} sont respectivement, indépendamment les uns des autres, un groupe méthyle, éthyle, phényle, un atome d'halogène, COOR^{3,1,1}, CONR^{3,1,1}R^{3,1,2}, CN, NR^{3,1,1}R^{3,1,2}, NHCOR^{3,1,1}, OR^{3,2,1}, O-halogénoalkyle en C₁ à C₃, SO₂R^{3,1,1}, SO₂NH₂, halogénoalkyle en C₁ à C₃, R^{3,2,1} et R^{3,1,2} pouvant être indépendamment l'un de l'autre, H, un groupe méthyle, éthyle, propyle
R³,² est H ou un groupe méthyle
est leurs sels, diastéréoméres, énantiomères, racémates, hydrates ou solvates pharmacologiquement acceptables.

14. Composés de formule 1 selon l'une des revendications 1 à 13, dans laquelle
R¹ est un groupe azétidine,
R² est un groupe pipérazine,
R³ est un groupe O-alkylène en C₁ à C₂-phényle ou NH-alkylène en C₁ à C₂-phényle
et
R⁴ est Cl et leurs sels, diastéréomères, énantiomères, racémates, hydrates ou solvates pharmacologiquement acceptables.

15. Composés de formule 2 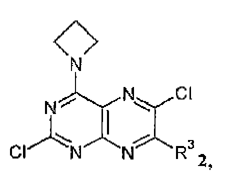 dans laquelle
R³ est un groupe de formule 1a 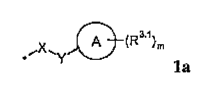 dans laquelle
A est un cycle choisi dans le groupe comprenant un cycle monocyclique, hétérocyclique, un cycle bicyclique qui contient éventuellement un ou plusieurs hétéroatomes, un groupe aryle en C₆ à C₁₀ et un groupe hétéroaryle en C₅ à C₁₀
X est NR^{3,2}, O, S ;
Y est un groupe alkyléne en C₁ à C₄ qui peut être substitué éventuellement par un ou plusieurs R^{3,3}
m désigne 0, 1, 2 ou 3,
les R^{3,1}, respectivement, indépendamment les uns des autres sont choisies dans un groupe comprenant les groupes alkyle en C₁ à C₆, aryle en C₆ à C₁₀, COOR^{3,1,1}, CONR^{3,1,1}R^{l3,1,2}, CN, NR^{3,1,1}R^{3,1,2}, NHCOR^{3,1,1}, OR^{3,1,1}, O-halogénoalkyle en C₁ à C₆, SO₂R^{3,1,1}, SO₂NH₂, un atome d'halogène, un groupe halogénoalkyle en C₁ à C₆, alkyle en C₁ à C₆-CONR^{3,1,1}R^{3,1,2}, alkyle en C₁ à C₆-NR^{3,1,1}R^{3,1,2}, alkyle en C₁ à C₆-CONH₂, O-alkylène en C₁ à C₆-NH₂, O-cycloalkyle en C₃ à C₆, O-alkylène en C₁ à C₄-cycloalkyle en C₃ à C₆, O-alkylène en C₁ à C₄-CONH₂, SONR^{3,1,1}R^{3,1,2};
ou
dans laquelle
R^{3,1} forme conjointement avec deux atomes de A, un cycle carbocyclique de 5 ou 6 chaînons ou un cycle hétérocyclique de 5 à 6 chaînons qui peut contenir éventuellement un ou plusieurs hétéroatomes choisis dans le groupe comprenant un atome d'oxygène et d'azote,
dans laquelle
R^{3,1,1} est H, un groupe alkyle en C₁ à C₆ ;
R^{3,1,2} est H, un groupe alkyle en C₁ à C₆ ;
et
R^{3,2} est H, un groupe alkyle en C₁ à C₆ ;
et dans laquelle
les R^{3,3} sont respectivement, indépendamment les uns des autres, H, un groupe alkyle en C₁ à C₆, alkyle en C₁ à C₆-OH, cycloalkyle en C₃ à C₆, cycloalkyle en C₃ à C₆-OH, O-alkyle en C₁ à C₆, COOR^{3,1,1}, COO-alkyle en C₁ à C₆, ou CONR^{3,1,1}R^{3,1,2} ;
ou dans laquelle
R^{3,3} forme conjointement avec un ou deux atomes de carbone de Y, un cycle carbocyclique comportant 3, 4, 5 ou 6 atomes de carbone, et leurs sels, diastéréomères, énantiomères, racémates, hydrates ou solvates pharmaoologiquement acceptables.

16. Médicament contenant l'un des composés selon l'une des revendications 1 à 14.

17. Utilisation de composés selon l'une des revendications 1 à 14, pour la production d'un médicament destiné au traitement de troubles ou de maladies des voies respiratoires ou gastro-intestinaux et également de maladies inflammatoires des articulations, de la peau ou des yeux, les cancers et les maladies du système nerveux périphérique et central.

18. Utilisation de composés selon l'une des revendications 1 à 14 pour la production d'un médicament destiné au traitement de maladies inflammatoires et obstructives telles que la BPCO, la sinusite chronique, l'asthme, la maladie de Crohn, la rectocolite hémorragique.

19. Utilisation selon l'une des revendications 17 à 18, dans laquelle les effets secondaires du traitement sont réduits.

20. Utilisation selon l'une des revendications 17 à 18, dans laquelle les effets secondaires réduits sont choisis dans le groupe comprenant les vomissements et les nausées.
